# EUROPEAN PATENT APPLICATION

(11) **EP 3 245 865 A1**
(43) Date of publication of application: **22.11.2017**
(21) Application number: 16170036.4
(22) Date of filing: 17.05.2016
(51) Int. Cl.: A01H 5/10, C12N 15/82

(54) **METHOD FOR INCREASING YIELD IN BRASSICACEAE**

(71) Applicant: Bayer CropScience Aktiengesellschaft, 40789 Monheim am Rhein (DE); Bayer CropScience NV, 1831 Diegem (BE)
(72) Inventor: The designation of the inventor has not yet been filed
(74) Representative: BIP Patents

(57) **Abstract**

The invention relates to a method for increasing yield in *Brassicaceae* comprising at least one event or allele as described, wherein that the *Brassicaceae* plants, plant parts thereof, plant propagation material or the soil in which *Brassicaceae* plants are grown or intended to be grown are treated with the active ingredients or mixtures thereof.

## Description

The invention relates to a method for increasing yield in *Brassicaceae* comprising at least one event or allele as described, wherein that the *Brassicaceae* plants, plant parts thereof, plant propagation material or the soil in which *Brassicaceae* plants are grown or intended to be grown are treated with the active ingredients or mixtures thereof.

*Brassicaceae* represent an economically important family of flowering plants also called Cruciferae due to the cross-like shape of the flower or cabbage plants. Important economic species are found in vegetables, e.g. Brassica oleracea (broccoli, cabbage, cauliflower, etc.), Brassica rapa (turnip, Chinese cabbage, etc.), oil crops, e.g. Brassica napus (rapeseed, Canola, etc.), ornamentals (Wallflower, Alyssum) or as model crops like Arabidopsis thaliana (thale cress) which is intensively investigated. Rapeseed oil is currently one of the most used cooking oils and is produced at over 18 millions metric tons for food and industrial use after palm oil and soybean oil. Therefore there is an ongoing interest to further secure and increase yield in *Brassicaceae* crops.

It has now been found that, surprisingly, certain active ingredients or mixtures thereof are particularly suitable for increasing yield in *Brassicaceae,* in particular in winter and spring oilseed rape or Canola.

The following active ingredients active ingredients can be used to treat *Brassicaceae* plants or parts thereof, including seeds, to increase yield:

### Herbicides:

Herbicidally active compounds useful in the context of the present invention, also in mixed formulations or in tank mix, are, for example, known active compounds as they are described in, for example, Weed Research 26, 441-445 (1986), or "The Pesticide Manual", 16th edition, November 2012, The British Crop Protection Council and the Royal Soc. of Chemistry, and the literature cited therein, and which for example act as inhibitor of acetolactate synthase, acetyl-CoA-carboxylase, cellulose-synthase, enolpyruvylshikimat-3-phosphat-synthase, glutamin-synthetase, p-hydroxyphenylpyruvat-dioxygenase, phytoendesaturase, photosystem I, photosystem II, and/or protoporphyrinogen-oxidase.

Examples of active compounds which may be mentioned as herbicides or plant growth regulators which are known from the literature and which can be combined with the compounds according to the invention are the following (compounds are either described by "common name" in accordance with the International Organization for Standardization (ISO) or by chemical name or by a customary code number), and always comprise all applicable forms such as acids, salts, ester, or modifications such as isomers, like stereoisomers and optical isomers. As an example at least one applicable form and/or modifications can be mentioned.

Examples for **herbicides** useful in the context of the present invention are disclosed in group H-1: Acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate, and - octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenac-sodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, -dimethylammonium, -diolamin, -ethyl, -2-ethylhexyl, -isobutyl, -isooctyl, -isopropylammonium, -potassium, -triisopropanolammonium, and -trolamine, 2,4-DB, 2,4-DB-butyl, -dimethylammonium, -isooctyl, -potassium, and -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-{2-chloro-4-fluoro-5-[4-(3-fluoropropyl)-5-oxo-4,5-dihydro-1H-tetrazol-1-yl]phenyl}ethanesulfonamide, F-7967, i. e. 3-[7-chloro-5-fluoro-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and -methyl, fluoroglycofen, fluoroglycofen-ethyl, flu-propanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, -potassium, -sodium, and -trimesium, H-9201, i.e. O-(2,4-dimethyl-6-nitrophenyl) O-ethyl isopropylphosphoramidothioate, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl) ethyl-(2,4-dichlorophenoxy)acetate, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, -potassium and -sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl, -dimethylammonium, -2-ethylhexyl, -isopropylammonium, -potassium, and -sodium, MCPB, MCPB-methyl, -ethy,1 and -sodium, mecoprop, mecoprop-sodium, and -butotyl, mecoprop-P, mecoprop-P-butotyl, -dimethylammonium, -2-ethylhexyl, and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-(3-chloro-4-isopropylphenyl)-2-methylpentan amide, NGGC-011, napropamide, NC-310, i.e. [5-(benzyloxy)-1-methyl-1H-pyrazol-4-yl](2,4-dichlorophenyl)methanone, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrione, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trichloroacetic acid), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-dichloro-N-{2-[(4,6-dimethoxypyrimidin-2-yl)oxy]benzyl}aniline, and the following compounds:

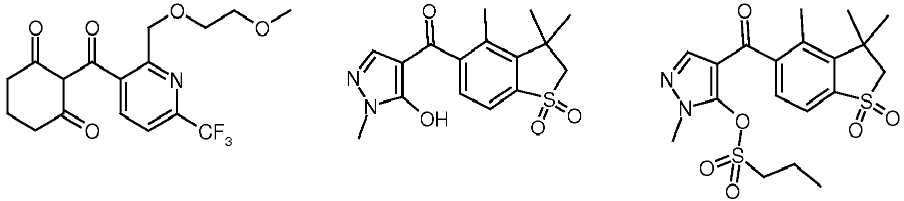

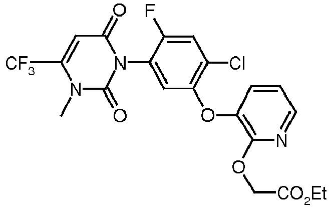

Group H-2 discloses herbicides especially used in the context of the present invention are selected from the group consisting of:
Acetochlor, Alachlor, Dicamba, Diflufenican, Flufenacet, Fluroxypyr, Foramsulfuron, Glufosinate, L-Glufosinate, Glyphosate, Hexazinone, Imazamox, Imazethapyr, Indaziflam, Iodosulfuron, Isoxaflutole, Mesosulfuron, Mesotrione, Metolachlor, Metribuzin, Metsulfuron, Pendimethalin, Penoxsulam, Picloram, Pinoxaden, Pyroxsulam, Quinmerac, Rimsulfuron, Thiencarbazone, Tembotrione, Thifensulfuron, and/or Tribenuron, and the esters and/or agronomically acceptable salts of these herbicides.

Group H-3 discloses preferred herbicides are selected from the group consisting of:
Acetochlor, Dicamba, Diflufenican, Flufenacet, Foramsulfuron, Glufosinate, L-Glufosinate, Glyphosate, Iodosulfuron, Isoxaflutole, Mesosulfuron, Mesotrione, Metribuzin, Pinoxaden, Tembotrione, and /or Thiencarbazone, and the esters and/or agronomically acceptable salts of these herbicides.

If Glyphosate salts are used as herbicide, preference is given to Glyphosate isopropylamine salt, Glyphosate potassium salt, Glyphosate sodium salt, Glyphosate trimethylsulfonium salt.

If (L-)Glufosinate salts are used as herbicide, preference is given to (L-)Glufosinate-ammonium salt, (L-)Glufosinate potassium salt and (L-)Glufosinate sodium salt.

If Foramsulfuron salts are used as herbicide, preference is given to Foramsulfuron-sodium.

If Iodosulfuron esters and/or salts thereof are used as herbicide, preference is given to Iodosulfuron-methyl and Iodosulfuron-methyl-sodium.

If Mesosulfuron esters and/or salts thereof are used as herbicide, preference is given to Mesosulfuron-methyl and Mesosulfuron-methyl-sodium.

If Thiencarbazone esters and/or salts thereof are used as herbicide, preference is given to Thiencarbazone-methyl and Thiencarbazone-methyl-sodium.

Preferred mixtures of herbicides used in the context of the present invention are disclosed in group H-4: mixtures of Acetochlor and other herbicides mentioned herein, mixtures of Dicamba, esters and/or salts thereof and other herbicides mentioned herein, mixtures of Diflufenican and other herbicides mentioned herein, mixtures of Flufenacet and other herbicides mentioned herein, mixtures of Glufosinate and/or salts thereof (preferably Glufosinate-ammonium salt, Glufosinate potassium salt and Glufosinate sodium salt) and other herbicides mentioned herein, mixtures of L-Glufosinate and/or salts thereof (preferably L-Glufosinate-ammonium salt, L-Glufosinate potassium salt and L-Glufosinate sodium salt) and other herbicides mentioned herein, mixtures of Indaziflam and other herbicides mentioned herein, mixtures of Isoxaflutole and other herbicides mentioned herein, mixtures of Mesosulfuron esters and/or salts thereof (preferably Mesosulfuron-methyl(-sodium)) and other herbicides mentioned herein, mixtures of Metribuzin and other herbicides mentioned herein, mixtures of Thiencarbazone esters and/or salts thereof (preferably Thiencarbazone-methyl(-sodium)) and other herbicides mentioned herein, mixtures of Tembotrione and other herbicides mentioned herein.

Particularly preferred mixtures of herbicides used in the context of the present invention are disclosed in group H-5:
mixtures of Acetochlor and Isoxaflutole, mixtures of Acetochlor and Tembotrione mixtures of Diflufenican and Flufenacet, mixtures of Diflufenican and Metribuzin, mixtures of Glufosinate and/or salts thereof and Indaziflam, in particular mixtures of Glufosinate-ammonium and Indaziflam, mixtures of L-Glufosinate and/or salts thereof and Indaziflam, in particular mixtures of L-Glufosinate-ammonium and Indaziflam, mixtures of Mesosulfuron-methyl(-sodium) and Iodosulfuron-methyl(-sodium), mixtures of Mesosulfuron-methyl(-sodium), Iodosulfuron-methyl(-sodium) and Flufenacet, mixtures of Tembotrione and Isoxaflutole, mixtures of Tembotrione, Isoxaflutole, and Acetochlor.

Examples for **plant growth regulators** useful in the context of the present invention are disclosed in group P-1:
Acibenzolar, acibenzolar-S-methyl, 5-aminolevulinic acid, ancymidol, 6-benzylaminopurine, Brassinolid, catechine, chlormequat chloride, cloprop, cyclanilide, 3-(cycloprop-1-enyl) propionic acid, daminozide, dazomet, n-decanol, dikegulac, dikegulac-sodium, endothal, endothal-dipotassium, -disodium, and -mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, jasmonic acid, maleic hydrazide, mepiquat chloride, 1-methylcyclopropene, methyl jasmonate, 2-(1-naphthyl)acetamide, 1-naphthylacetic acid, 2- naphthyloxyacetic acid, nitrophenolate-mixture, paclobutrazol, N-(2-phenylethyl)-beta-alanine, N-phenyl-phthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

Examples for **safeners** useful in the context of the present invention are disclosed in group SAF-1 in 16 subgroups:
S1) compounds of the group of heterocyclic carboxylic acid derivatives:
S1^{a}) compounds of the type of dichlorophenylpyrazoline-3-carboxylic acid (S1^{a}), preferably compounds such as 1-(2,4-dichlorophenyl)-5-(ethoxycarbonyl)-5-methyl-2-pyrazoline-3-carboxylic acid, ethyl 1-(2,4-dichlorophenyl)-5-(ethoxycarbonyl)-5-methyl-2-pyrazoline-3-carboxylate (S1-1) ("mefenpyr(-diethyl)"), and related compounds, as described in WO-A-91/07874;
S1^{b}) derivatives of dichlorophenylpyrazolecarboxylic acid (S1^{b}) preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4) and related compounds, as described in EP-A-333 131 and EP-A-269 806;
S1^{c}) derivatives of 1,5-diphenylpyrazole-3-carboxylic acid (S1^{c}), preferably compounds such as ethyl 1-(2,4-dichlorophenyl)-5-phenylpyrazole-3-carboxylate (S1-5), methyl 1-(2-chlorophenyl)-5-phenylpyrazole-3-carboxylate (S1-6) and related compounds, as described, for example, in EP-A-268554;
S1^{d}) compounds of the type of triazolecarboxylic acids (S1^{d}), preferably compounds such as fenchlorazole(-ethyl), i.e. ethyl 1-(2,4-dichlorophenyl)-5-trichloromethyl-(1H)-1,2,4-triazole-3-carboxylate (S1-7), and related compounds, as described in EP-A-174 562 and EP-A-346 620;
S1^{e}) compounds of the type of 5-benzyl- or 5-phenyl-2-isoxazoline-3-carboxylic acid or 5,5-diphenyl-2-isoxazoline-3-carboxylic acid (S1^{e}), preferably compounds such as ethyl 5-(2,4-dichlorobenzyl)-2-isoxazoline-3-carboxylate (S1-8) or ethyl 5-phenyl-2-isoxazoline-3-carboxylate (S1-9) and related compounds, as described in WO-A-91/08202, or 5,5-diphenyl-2-isoxazolinecarboxylic acid (S1-10) or ethyl 5,5-diphenyl-2-isoxazolinecarboxylate (S1-11) ("isoxadifen-ethyl") or n-propyl 5,5-diphenyl-2-isoxazolinecarboxylate (S1-12) or ethyl 5-(4-fluorophenyl)-5-phenyl-2-isoxazoline-3-carboxylate (S1-13), as described in the patent application WO-A-95/07897;
S2) Compounds of the group of 8-quinolinoxy derivatives (S2):
S2^{a}) compounds of the type of 8-quinolinoxyacetic acid (S2^{a}), preferably 1-methylhexyl (5-chloro-8-quinolinoxy)acetate (common name "cloquintocet-mexyl" (S2-1), 1,3-dimethyl-but-1-yl (5-chloro-8-quinolinoxy)acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl (5-chloro-8-quinolinoxy)acetate (S2-6), allyl (5-chloro-8-quinolinoxy)acetate (S2-7), 2-(2-propylideneiminoxy)-1-ethyl (5-chloro-8-quinolinoxy)acetate (S2-8), 2-oxo-prop-1-yl (5-chloro-8-quinolinoxy)acetate (S2-9) and related compounds, as described in EP-A-86 750, EP-A-94 349 and EP-A-191 736 or EP-A-0 492 366, and also (5-chloro-8-quinolinoxy)acetic acid (S2-10), its hydrates and salts, for example its lithium, sodium, potassium, calcium, magnesium, aluminium, iron, ammonium, quaternary ammonium, sulphonium or phosphonium salts, as described in WO-A-2002/34048;
S2^{b}) compounds of the type of (5-chloro-8-quinolinoxy)malonic acid (S2^{b}), preferably compounds such as diethyl (5-chloro-8-quinolinoxy)malonate, diallyl (5-chloro-8-quinolinoxy)malonate, methyl ethyl (5-chloro-8-quinolinoxy)malonate and related compounds, as described in EP-A-0 582 198;
S3) Active compounds of the type of dichloroacetamides (S3) which are frequently used as pre-emergence safeners (soil-acting safeners), such as, for example, "dichlormid" (N,N-diallyl-2,2-dichloroacetamide) (S3-1), "R-29148" (3-dichloroacetyl-2,2,5-trimethyl-1,3-oxazolidine) from Stauffer (S3-2), "R-28725" (3-dichloroacetyl-2,2-dimethyl-1,3-oxazolidine) from Stauffer (S3-3), "benoxacor" (4-dichloroacetyl-3,4-dihydro-3-methyl-2H-1,4-benzoxazine) (S3-4), "PPG-1292" (N-allyl-N-[(1,3-dioxolan-2-yl)methyl]dichloroacetamide) from PPG Industries (S3 5),
   "DKA-24" (N-allyl-N-[(allylaminocarbonyl)methyl]dichloroacetamide) from Sagro-Chem (S3-6),
   "AD-67" or "MON 4660" (3-dichloroacetyl-1-oxa-3-aza-spiro[4,5]decane) from Nitrokemia or Monsanto (S3-7), "TI-35" (1-dichloroacetylazepane) from TRI-Chemical RT (S3-8) "diclonon" (dicyclonon) or "BAS145138" or "LAB145138" (S3-9) ((RS)-1-dichloroacetyl-3,3,8a-trimethylperhydropyrrolo[1,2-a]pyrimidin-6-one) from BASF, furilazole" or "MON 13900" ((RS)-3-dichloroacetyl-5-(2-furyl)-2,2-dimethyloxazolidine) (S3-10), and also its (R)-isomer (S3-11);
S4) Compounds of the class of acylsulphonamides (S4):
S4^{a}) N-acylsulphonamides of the formula (S4^{a}) and salts thereof, as described in WO-A-97/45016 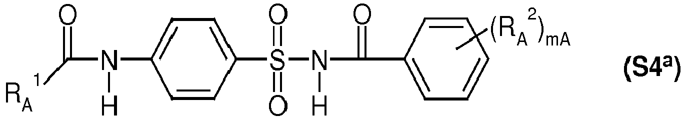 in which
   - R_{A}¹: is (C₁-C₆)-alkyl, (C₃-C₆)-cycloalkyl, where the 2 last-mentioned radicals are substituted by v_{A} substituents from the group consisting of halogen, (C₁-C₄)-alkoxy, halo-(C₁-C₆)-alkoxy and (C₁-C₄)-alkylthio and, in the case of cyclic radicals, also (C₁-C₄)-alkyl and (C₁-C₄)-haloalkyl;
   - R_{A}²: is halogen, (C₁-C₄)-alkyl, (C₁-C₄)-alkoxy, CF₃;
   - m_{A}: is 1 or 2;
   - v_{D}: is 0, 1, 2 or 3;
S4^{b}) compounds of the type of 4-(benzoylsulphamoyl)benzamides of the formula (S4^{b}) and salts thereof, as described in WO-A-99/16744, 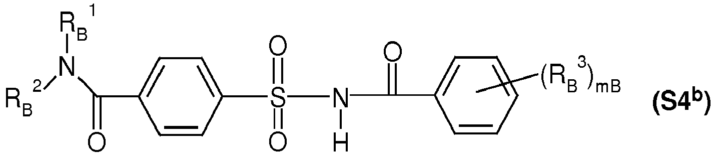 in which
   - R_{B}¹, R_{B}²: independently of one another are hydrogen, (C₁-C₆)-alkyl, (C₃-C₆)-cycloalkyl, (C₃-C₆)-alkenyl, (C₃-C₆)-alkynyl,
   - R_{B}³: is halogen, (C₁-C₄)-alkyl, (C₁-C₄)-haloalkyl or (C₁-C₄)-alkoxy,
   - m_{B}: is 1 or 2;
   for example those in which
   R_{B}¹ = cyclopropyl, R_{B}²= hydrogen and (R_{B}³) = 2-OMe ("cyprosulfamide", S4-1),
   R_{B}¹ = cyclopropyl, R_{B}²= hydrogen and (R_{B}³) = 5-Cl-2-OMe (S4-2),
   R_{B}¹ = ethyl, R_{B}²= hydrogen and (R_{B}³) = 2-OMe (S4-3),
   R_{B}¹ = isopropyl, R_{B}²= hydrogen and (R_{B}³) = 5-Cl-2-OMe (S4-4) and
   R_{B}¹= isopropyl, R_{B}²= hydrogen and (R_{B}³) = 2-OMe (S4-5);
S4^{c}) compounds of the class of benzoylsulphamoylphenylureas of the formula (S4^{c}) as described in EP-A-365484, 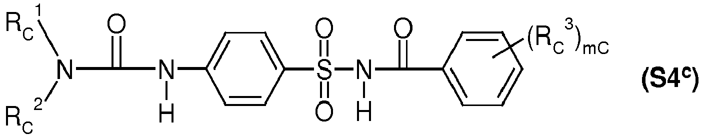 in which
   - R_{C}¹, R_{C}²: independently of one another are hydrogen, (C₁-C₈)-alkyl, (C₃-C₈)-cycloalkyl, (C₃-C₆)-alkenyl, (C₃-C₆)-alkynyl,
   - R_{C}³: is halogen, (C₁-C₄)-alkyl, (C₁-C₄)-alkoxy, CF₃,
   - m_{C}: is 1 or 2;
   for example
   1-[4-(N-2-methoxybenzoylsulphamoyl)phenyl]-3-methylurea,
   1-[4-(N-2-methoxybenzoylsulphamoyl)phenyl]-3,3-dimethylurea,
   1-[4-(N-4,5-dimethylbenzoylsulphamoyl)phenyl]-3-methylurea;
S4^{d}) compounds of the type of N-phenylsulphonylterephthalamides of the formula (S4^{d}) and salts thereof, which are known, for example, from CN 101838227, 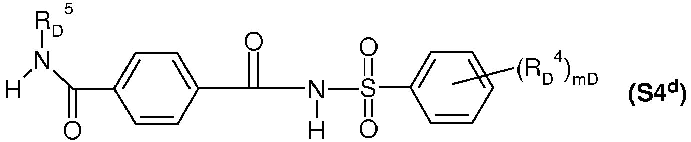 in which
   R_{D}⁴ is halogen, (C₁-C₄)-alkyl, (C₁-C₄)-alkoxy, CF₃;
   m_{D} is 1 or 2;
   R_{D}⁵ is hydrogen, (C₁-C₆)-alkyl, (C₃-C₆)-cycloalkyl, (C₂-C₆)-alkenyl, (C₂-C₆)-alkynyl, (C₅-C₆)-cycloalkenyl;
S5) Active compounds from the class of hydroxyaromatics and aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 2,4-dichlorocinnamic acid, as described in WO-A-2004/084631, WO-A-2005/015994, WO-A-2005/016001;
S6) Active compounds from the class of 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-(2-methylsulphonylaminoethyl)-3-(2-thienyl)-1,2-dihydroquinoxalin-2-one, as described in WO-A-2005/112630;
S7) Compounds from the class of diphenylmethoxyacetic acid derivatives (S7), for example methyl diphenylmethoxyacetate (CAS-Reg.Nr. 41858-19-9) (S7-1), ethyl diphenylmethoxyacetate, or diphenylmethoxyacetic acid, as described in WO-A-98/38856;
S8) Compounds of the formula (S8), as described in WO-A-98/27049, 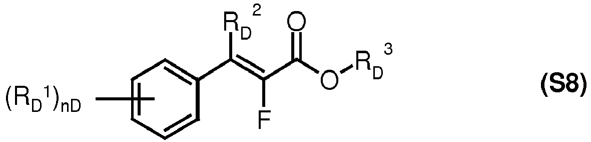 where the symbols and indices have the following meanings:
   R_{D}1 is halogen, (C₁-C₄)-alkyl, (C₁-C₄)-haloalkyl, (C₁-C₄)-alkoxy, (C₁-C₄)-haloalkoxy,
   R_{D}² is hydrogen or (C₁-C₄)-alkyl,
   R_{D}³ is hydrogen, (C₁-C₈)-alkyl, (C₂-C₄)-alkenyl, (C₂-C₄)-alkynyl or aryl, where each of the carbon-containing radicals mentioned above is unsubstituted or substituted by one or more, preferably by up to three, identical or different radicals from the group consisting of halogen and alkoxy; or salts thereof,
   n_{D} is an integer from 0 to 2;
S9) Active compounds from the class of 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No.: 219479-18-2), 1,2-dihydro-4-hydroxy-1-methyl-3-(5-tetrazolylcarbonyl)-2-quinolone (CAS Reg. No.: 95855-00-8), as described in WO-A-1999/000020;
S10) Compounds of the formula (S10^{a}) or (S10^{b}) as described in WO-A-2007/023719 and WO-A-2007/023764 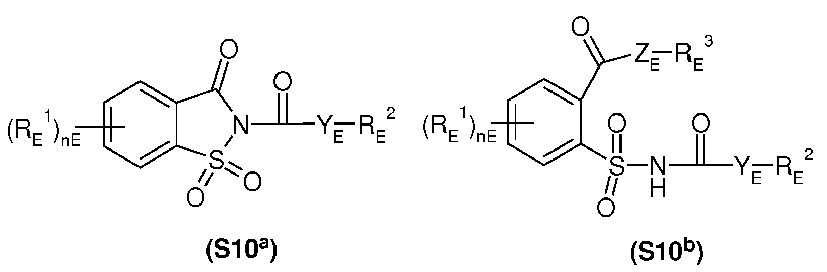 in which
   - R_{E}1: is halogen, (C₁-C₄)-alkyl, methoxy, nitro, cyano, CF₃, OCF₃
   - Y_{E}, Z_{E}: independently of one another are O or S,
   - n_{E}: is an integer from 0 to 4,
   - R_{E}²: is (C₁-C₁₆)-alkyl, (C₂-C₆)-alkenyl, (C₃-C₆)-cycloalkyl, aryl; benzyl, halobenzyl,
   - R_{E}³: is hydrogen or (C₁-C₆)-alkyl;
S11) Active compounds of the type of oxyimino compounds (S11), which are known as seed dressings, such as, for example, "oxabetrinil" ((Z)-1,3-dioxolan-2-ylmethoxyimino(phenyl)acetonitrile) (S11-1), which is known as seed dressing safener for millet against metolachlor damage,
   "fluxofenim" (1-(4-chlorophenyl)-2,2,2-trifluoro-1-ethanone O-(1,3-dioxolan-2-ylmethyl)oxime) (S11-2), which is known as seed dressing safener for millet against metolachlor damage, and
   "cyometrinil" or "CGA-43089" ((Z)-cyanomethoxyimino(phenyl)acetonitrile) (S11-3), which is known as seed dressing safener for millet against metolachlor damage;
S12) Active compounds from the class of isothiochromanones (S12), such as, for example, methyl [(3-oxo-1H-2-benzothiopyran-4(3H)-ylidene)methoxy]acetate (CAS Reg. No.: 205121-04-6) (S12-1) and related compounds from WO-A-1998/13361;
S13) One or more compounds from group (S13):
   "naphthalic anhydrid" (1,8-naphthalenedicarboxylic anhydride) (S13-1), which is known as seed dressing safener for corn against thiocarbamate herbicide damage,
   "fenclorim" (4,6-dichloro-2-phenylpyrimidine) (S13-2), which is known as safener for pretilachlor in sown rice,
   "flurazole" (benzyl 2-chloro-4-trifluoromethyl-1,3-thiazole-5-carboxylate) (S13-3), which is known as seed dressing safener for millet against alachlor and metolachlor damage,
   "CL 304415" (CAS Reg. No.: 31541-57-8) (4-carboxy-3,4-dihydro-2H-1-benzopyran-4-acetic acid) (S13-4) from American Cyanamid, which is known as safener for corn against imidazolinone damage,
   "MG 191" (CAS Reg. No.: 96420-72-3) (2-dichloromethyl-2-methyl-1,3-dioxolane) (S13-5) from Nitrokemia, which is known as safener for corn,
   "MG 838" (CAS Reg. No.: 133993-74-5) (2-propenyl 1-oxa-4-azaspiro[4.5]decane-4-carbodithioate) (S13-6) from Nitrokemia,
   "disulphoton" (O,O-diethyl S-2-ethylthioethyl phosphorodithioate) (S13-7),
   "dietholate" (O,O-diethyl O-phenyl phosphorothioate) (S13-8),
   "mephenate" (4-chlorophenyl methylcarbamate) (S13-9);
S14) Active compounds which, besides a herbicidal effect against harmful plants, also have a safener effect on crop plants such as rice, such as, for example, "dimepiperate" or "MY 93" (*S*-1-methyl-1-phenylethyl piperidine-1-carbothioate), which is known as safener for rice against molinate herbicide damage,
   "daimuron" or "SK 23" (1-(1-methyl-1-phenylethyl)-3-p-tolylurea), which is known as safener for rice against imazosulphuron herbicide damage,
   "cumyluron" = "JC 940" (3-(2-chlorophenylmethyl)-1-(1-methyl-1-phenylethyl)urea, see JP-A-60087254), which is known as safener for rice against some herbicide damage,
   "methoxyphenone" or "NK 049" (3,3'-dimethyl-4-methoxybenzophenone), which is known as safener for rice against some herbicide damage,
   "CSB" (1-bromo-4-(chloromethylsulphonyl)benzene) from Kumiai (CAS Reg. No. 54091-06-4), which is known as safener against some herbicide damage in rice;
S15) Compounds of the formula (S15) or its tautomers, 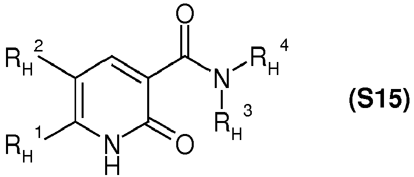 as described in WO-A-2008/131861 and WO-A-2008/131860, in which
   - R_{H}¹: is (C₁-C₆)-haloalkyl,
   - R_{H}²: is hydrogen or halogen,
   - R_{H}³, R_{H}⁴: independently of one another are hydrogen, (C₁-C₁₆)-alkyl, (C₂-C₁₆)-alkenyl or (C₂-C₁₆)-alkynyl,
   where each of the 3 last-mentioned radicals is unsubstituted or substituted by one or more radicals from the group consisting of halogen, hydroxy, cyano, (C₁-C₄)-alkoxy, (C₁-C₄)-haloalkoxy, (C₁-C₄)-alkylthio, (C₁-C₄)-alkylamino, di-[(C₁-C₄)-alkyl]-amino, [(C₁-C₄)-alkoxy]-carbonyl, [(C₁-C₄)-haloalkoxy]-carbonyl, unsubstituted or substituted (C₃-C₆)-cycloalkyl, unsubstituted or substituted phenyl, and unsubstituted or substituted heterocyclyl;
   or (C₃-C₆)-cycloalkyl, (C₄-C₆)-cycloalkenyl, (C₃-C₆)-cycloalkyl which is at one site of the ring condensed with a 4 to 6-membered saturated or unsaturated carbocyclic ring, or (C₄-C₆)-cycloalkenyl which is at one site of the ring condensed with a 4 to 6-membered saturated or unsaturated carbocyclic ring,
   where each of the 4 last-mentioned radicals is unsubstituted or substituted by one or more radicals from the group consisting of halogen, hydroxy, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di-(C₁-C₄)-alkyl]-amino, [(C₁-C₄)-alkoxy]-carbonyl, [(C₁-C₄)-haloalkoxy]-carbonyl, unsubstituted or substituted (C₃-C₆)-cycloalkyl, unsubstituted or substituted phenyl, and unsubstituted or substituted heterocyclyl; or
   - R_{H}³: is (C₁-C₄)-alkoxy, (C₂-C₄)-alkenyloxy, (C₂-C₆)-alkynyloxy or (C₂-C₄)-haloalkoxy, and
   - R_{H}⁴: is hydrogen or (C₁-C₄)-alkyl, or
   - R_{H}³ and R_{H}⁴: together with the directly bound N-atom are a 4 to 8-membered heterocyclic ring, which can contain further hetero ring atoms besides the N-atom, preferably up to two further hetero ring atoms from the group consisting of N, O and S, and which is unsubstituted or substituted by one or more radicals from the group consisting of halogen, cyano, nitro, (C₁-C₄)-alkyl, (C₁-C₄)-haloalkyl, (C₁-C₄)-alkoxy, (C₁-C₄)-haloalkoxy, and (C₁-C₄)-alkylthio;
S16) Active compounds which are primarily used as herbicides, but also have a safener effect on crop plants, for example
   (2,4-dichlorophenoxy) acetic acid (2,4-D), (4-chlorophenoxy) acetic acid, (R,S)-2-(4-chloro-o-tolyloxy)propionic acid (mecoprop), 4-(2,4-dichlorophenoxy)butyric acid (2,4-DB), (4-chloro-o-tolyloxy)acetic acid (MCPA), 4-(4-chloro-o-tolyloxy)butyric acid, 4-(4-chlorophenoxy)butyric acid, 3,6-dichloro-2-methoxybenzoic acid (dicamba), 1-(ethoxycarbonyl)ethyl 3,6-dichloro-2-methoxybenzoate (lactidichlor-ethyl).

Preferred safeners in the context of the present invention are disclosed in group SAF-2: cloquintocet-mexyl, cyprosulfamid, fenchlorazole-ethyl, isoxadifen-ethyl, mefenpyr-diethyl, fenclorim, cumyluron, S4-1, and S4-5, wherein particular preference is given to the safeners cloquintocet-mexyl, cyprosulfamid, isoxadifen-ethyl, and mefenpyr-diethyl.

Particularly preferred safeners used in the context of the present invention are disclosed in group SAF-3: Isoxadifen and its esters, in particular Isoxadifen-ethyl, and Cyprosulfamide.

The safeners are preferably used together with one or more of the herbicides mentioned above. Preferred in the context of the present invention are mixtures of safeners disclosed in group SAF-4: mixtures of Isoxadifen-ethyl and the herbicides mentioned herein and mixtures of Cyprosulfamide and the herbicides mentioned herein.

The herbicides and safeners mentioned above are known, see e.g. "The Pesticide Manual", 16th edition, November 2012, The British Crop Protection Council and the Royal Soc. of Chemistry.

The herbicides and the mixtures of herbicides (and optionally safeners) mentioned herein may be used in pre-emergence applications and/or in post-emergence applications.

In accordance with the present invention, the herbicides and the mixtures of herbicides (and optionally safeners) mentioned herein may be applied as a split application over time. Another possibility is the application of the individual active ingredients or the mixtures comprising the active ingredients in a plurality of portions (sequential application), for example after pre-emergence applications, followed by post-emergence applications or after early post-emergence applications, followed by applications at medium or late post-emergence.

### Biologics:

Biologics as used in the context of the present invention are selected from the group B-1 consisting of:
*Bacillus cereus* EIP-N2 (Accession No. CNCM I-1562), *Myrothecium verrucaria* in particular strain AARC-0255, *Flavobacterium sp* strain H492 (Accession No. B-50584), *Bacillus subtilis* AQ713 (Accession No. B-21661), *Bacillus subtilis* AQ30002 (Accession No. B-50421), *Bacillus subtilis* AQ 30004 (Accession No. B-50455), *Bacilus firmus,* especially *Bacillus firmus* GB126 (Accession No. CNCM I-1582) and *Bacillus firmus* strain I-1582 (Accession No. CNCMI-1582), *Paecilomyces lilacinus* 251 (Accession No. DSM 23289), *Coniothyrium minitans* (Accession No. DSM 9660), *Bacillus pumilus* QST 2808 (Accession No. NRRL B-30087), *Bacillus pumilus* GB34 (Accession No. ATCC 700814), *Metarhizium anisopliae* (Accession No. DSM 3884), *Metarhizium anisopliae* (Accession No. ATCC 90448), *Bacillus subtilis*/*amyloliquefaciens* MBI600 (Accession No. NRRL B-50595), MBI600 + pyraclostrobin), *Paenibacillus polymyxa* strains Lu16774 (Accession No. DSM 26969), Lu17007 (Accession No. DSM 26970), and Lu17015 (Accession No. DSM 26971), *Bacillus sobtilis* FZB24 (EPA Registration Number: 72098-5 and EPA Establishment Number: 73386-DEU-001, a strain that is commercially available from Earth Bioscience, Inc.), *Pasteuria usage* strains (e.g., used in ECONEM), *Trichoderma virens* GI-3 (Accession No. ATCC 58678), *Bacillus amyloliquefaciens* TJ1000 (Accession No. ATCC BAA-390), or a combination of GI-3 with TJ1000 or FZB24, *Bacillus amyloliquefaciens* D747 (Accession No. FERM BP-8234), *Bradyrhizobium japonicum*, *Pseudomonas fluorescens, Pseudomonas chloropsis, Rhizobium* spp, especially *Rhizobium tropici* SP25, a recombinant exosporium-producing *Bacillus thuiringienses* cell (especially *B. thuringiensis* BT013A) that expresses a fusion protein comprising:
   1) an endoglucanase, (especially from *Bacillus subtilis*) having SEQ ID NO. 2 and
   2) a targeting sequence having at least about 43% identity with amino acids 20-35 of SEQ ID NO: 1, wherein the identity with amino acids 25-35 is at least about 54%
   and a recombinant exosporium-producing *Bacillus thuringiensis* cell that expresses a fusion protein comprising:
   1) a phosphoplipase having SEQ ID NO. 3 and
   2) a targeting sequence having at least about 43% identity with amino acids 20-35 of SEQ ID NO: 1, wherein the identity with amino acids 25-35 is at least about 54%.

The term "targeting sequence" as used herein refers to a polypeptide sequence that results in the localization of a longer polypeptide or the protein to the exosporium of a *Bacillus cereus* family member.

BclA, the major constituent of the surface nap, has been shown to be attached to the exosporium with its amino-terminus (N-terminus) positioned at the basal layer and its carboxy-terminus (C- terminus) extending outward from the spore. It was previously discovered that certain sequences from the N-terminal regions of BclA and BclB could be used to target a peptide or protein to the exosporium of a *Bacillus cereus* endospore (see, e.g., US Patent Publication Nos. 2010/0233124 and 2011/0281316, and Thompson, et al., "Targeting of the BclA and BclB Proteins to the Bacillus anthracis Spore Surface," Molecular Microbiology, 70(2):421-34 (2008), the entirety of each of which is hereby incorporated by reference). It was also found that the BetA/BAS3290 protein of Bacillus anthracis localized to the exosporium.

In particular, amino acids 20-35 of BclA from Bacillus anthracis Sterne strain have been found to be sufficient for targeting to the exosporium. A sequence alignment of amino acids 1-41 of BclA (SEQ ID NO: 1) with the corresponding N-terminal regions of several other Bacillus cereus family exosporium proteins and Bacillus cereus family proteins having related sequences is shown in WO 2016/044529 as Fig. 1. There is a region of high-homology among all of the proteins in the region corresponding to amino acids 20-41 of BclA. However, in these sequences, the amino acids corresponding to amino acids 36-41 of BclA contain secondary structure and are not necessary for fusion protein localization to the exosporium.

Sequences:
SEQ ID NO. 1: is identical with SEQ ID NO.: 1 of WO 2016/044529 referring to the amino acid sequence of AA 1-41 of BclA
SEQ ID NO. 2: is identical with SEQ ID NO.: 107 of WO 2016/044529 referring to the amino acid sequence of endonuclease
SEQ ID NO. 3: is identical with SEQ ID NO.: 108 of WO 2016/044529 referring to the amino acid sequence of B. thuringiensis BT013A phospholipase C

In one preferred embodiment, Biologics are selected from the group B-2 consisting of:
*Bacillus subtilis,* AQ713, *Bacilus firmus,* GB126, *Bacillus firmus* strain I-1582, *Paecilomyces lilacinus* 251, *Coniothyrium minitans* (Accession No. DSM 9660), *Bacillus pumilus* QST 2808, *Bacillus pumilus* GB34, *Metarhizium anisopliae* (Accession No. DSM 3884), *Metarhizium anisopliae* (Accession No. ATCC 90448), *Bacillus subtilis*/*amyloliquefaciens* MBI600, *Paenibacillus polymyxa* strains Lu16774, Lu17007, Lu17015, *Bacillus subtilis,* FZB24, *Pasteuria usage* strains (e.g. used in ECONEM),*Trichoderma virens* GI-3, *Bacillus amyloliquefaciens* TJ1000, a combination of GI-3 with TJ1000 or FZB24, *Bacillus amyloliquefaciens* D747, a recombinant exosporium-producing *Bacillus thuiringienses* cell (especially *B. thuringiensis* BT013A) that expresses a fusion protein comprising:
   1) an endoglucanase, (especially from *Bacillus subtilis*) having SEQ ID NO. 2 and
   2) a targeting sequence having at least about 43% identity with amino acids 20-35 of SEQ ID NO: 1, wherein the identity with amino acids 25-35 is at least about 54%
   and a recombinant exosporium-producing *Bacillus thuringiensis* cell that expresses a fusion protein comprising:
   1) a phosphoplipase having SEQ ID NO. 3 and
   2) a targeting sequence having at least about 43% identity with amino acids 20-35 of SEQ ID NO: 1, wherein the identity with amino acids 25-35 is at least about 54%.

In a more preferred embodiment, Biologics are selected from the group B-3 consisting of:
*Bacillus subtilis,* AQ713, *Bacillus firmus* GB126, *Paecilomyces lilacinus* strain 251, *Coniothyrium minitans* (Accession No. DSM 9660), *Bacillus pumilus* QST 2808, *Bacillus pumilus* GB34, a recombinant exosporium-producing *Bacillus thuiringienses* cell (especially *B. thuringiensis* BT013A) that expresses a fusion protein comprising:
   1) an endoglucanase, (especially from *Bacillus subtilis*) having SEQ ID NO. 2 and
   2) a targeting sequence having at least about 43% identity with amino acids 20-35 of SEQ ID NO: 1, wherein the identity with amino acids 25-35 is at least about 54%
   and a recombinant exosporium-producing *Bacillus thuringiensis* cell that expresses a fusion protein comprising:
   1) a phosphoplipase having SEQ ID NO. 3 and
   2) a targeting sequence having at least about 43% identity with amino acids 20-35 of SEQ ID NO: 1, wherein the identity with amino acids 25-35 is at least about 54%.

In a further more preferred embodiment, Biologics are selected from a group B-4 consisting of:
*Metarhizium anisopliae* (Accession No. DSM 3884), *Metarhizium anisopliae* (Accession No. ATCC 90448), *Bacillus subtilis*/*amyloliquefaciens* MBI600 (Accession No. NRRL B-50595), *Paenibacillus polymyxa* strains Lu16774, Lu17007, Lu17015, *Bacillus sobtilis* FZB24, *Pasteuria usage* strains (e.g. used in ECONEM)*,Trichoderma virens* GI-3, *Bacillus amyloliquefaciens* TJ1000, a combination of GI-3 with TJ1000 or FZB24) and *Bacillus amyloliquefaciens* D747.

In an even more preferred embodiment, Biologics are selected from the group B-5 consisting of:
*Bacillus sobtilis* AQ713, *Bacillus firmus* GB126, *Paecilomyces lilacinus* strain 251, *Coniothyrium minitans* (Accession No. DSM 9660), *Bacillus pumilus* GB34, and *Bacillus pumilus* QST 2808.

### Insecticides

The Insecticides and Nematicides identified herein by their common names are known and are described, for example, in the pesticide handbook ("The Pesticide Manual" 16th Ed., British Crop Protection Council 2012) or can be found on the Internet (e.g. http://www.alanwood.net/pesticides). The classification is based on the current IRAC Mode of Action Classification Scheme at the time of filing of this patent application.

Insecticides used in the context of the present invention are disclosed in the group I-1 comprising 29 subgroups:
(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion;
(2) GABA-gated chloride channel blockers, such as, for example, cyclodiene-organochlorines, for example chlordane and endosulfan or phenylpyrazoles (fiproles), for example ethiprole and fipronil;
(3) Sodium channel modulators, such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)- isomer)], tralomethrin and transfluthrin or DDT or methoxychlor;
(4) Nicotinic acetylcholine receptor (nAChR) competitive modulators, such as, for example, neonicotinoids, e.g. acetamiprid, clothianidin, dinotefuran, imidacloprid, nitenpyram, thiacloprid and thiamethoxam or nicotine or sulfoxaflor or flupyradifurone;
(5) Nicotinic acetylcholine receptor (nAChR) allosteric modulators, such as, for example, spinosyns, e.g. spinetoram and spinosad;
(6) Glutamate-gated chloride channel (GluCl) allosteric modulators, such as, for example, avermectins/milbemycins, for example abamectin, emamectin benzoate, lepimectin and milbemectin;
(7) Juvenile hormone mimics, such as, for example, juvenile hormone analogues, e.g. hydroprene, kinoprene and methoprene or fenoxycarb or pyriproxyfen;
(8) Miscellaneous non-specific (multi-site) inhibitors, such as, for example, alkyl halides, e.g. methyl bromide and other alkyl halides; or chloropicrine or sulphuryl fluoride or borax or tartar emetic or methyl isocyanate generators, e.g. diazomet and metam;
(9) Modulators of Chordotonal Organs, such as, for example pymetrozine or flonicamid;
(10) Mite growth inhibitors, such as, for example clofentezine, hexythiazox and diflovidazin or etoxazole;
(11) Microbial disruptors of the insect gut membrane, such as, for example Bacillus thuringiensis subspecies israelensis, Bacillus sphaericus, Bacillus thuringiensis subspecies aizawai, Bacillus thuringiensis subspecies kurstaki, Bacillus thuringiensis subspecies tenebrionis, and B.t. plant proteins: Cry1Ab, Cry1Ac, Cry1Fa, Cry1A.105, Cry2Ab, Vip3A, mCry3A, Cry3Ab, Cry3Bb, Cry34Ab1/35Ab1;
(12) Inhibitors of mitochondrial ATP synthase, such as, ATP disruptors such as, for example, diafenthiuron or organotin compounds, for example azocyclotin, cyhexatin and fenbutatin oxide or propargite or tetradifon;
(13) Uncouplers of oxidative phosphorylation via disruption of the proton gradient, such as, for example, chlorfenapyr, DNOC and sulfluramid;
(14) Nicotinic acetylcholine receptor channel blockers, such as, for example, bensultap, cartap hydrochloride, thiocylam, and thiosultap-sodium;
(15) Inhibitors of chitin biosynthesis, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron;
(16) Inhibitors of chitin biosynthesis, type 1, for example buprofezin;
(17) Moulting disruptor (in particular for Diptera, i.e. dipterans), such as, for example, cyromazine;
(18) Ecdysone receptor agonists, such as, for example, chromafenozide, halofenozide, methoxyfenozide and tebufenozide;
(19) Octopamine receptor agonists, such as, for example, amitraz;
(20) Mitochondrial complex III electron transport inhibitors, such as, for example, hydramethylnone or acequinocyl or fluacrypyrim;
(21) Mitochondrial complex I electron transport inhibitors, such as, for example from the group of the METI acaricides, e.g. fenazaquin, fenpyroximate, pyrimidifen, pyridaben, tebufenpyrad and tolfenpyrad or rotenone (Derris);
(22) Voltage-dependent sodium channel blockers, such as, for example indoxacarb or metaflumizone;
(23) Inhibitors of acetyl CoA carboxylase, such as, for example, tetronic and tetramic acid derivatives, e.g. spirodiclofen, spiromesifen and spirotetramat;
(24) Mitochondrial complex IV electron transport inhibitors, such as, for example, phosphines, e.g. aluminium phosphide, calcium phosphide, phosphine and zinc phosphide or cyanides, e.g. calcium cyanide, potassium cyanide and sodium cyanide;
(25) Mitochondrial complex II electron transport inhibitors, such as, for example, beta-ketonitrile derivatives, e.g. cyenopyrafen and cyflumetofen and carboxanilides, such as, for example, pyflubumide;
(28) Ryanodine receptor modulators, such as, for example, diamides, e.g. chlorantraniliprole, cyantraniliprole and flubendiamide,
(29) further active compounds such as, for example, Afidopyropen, Afoxolaner, Azadirachtin, Benclothiaz, Benzoximate, Bifenazate, Broflanilide, Bromopropylate, Chinomethionat, Chloroprallethrin, Cryolite, Cyclaniliprole, Cycloxaprid, Cyhalodiamide, Dicloromezotiaz, Dicofol, epsilon-Metofluthrin, epsilon-Momfluthrin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprole, Tetrachlorantraniliprole, Tioxazafen, Thiofluoximate, Triflumezopyrim and iodomethane; furthermore preparations based on Bacillus *firmus* (I-1582, BioNeem, Votivo), and also the following compounds: 1-{2-fluoro-4-methyl-5-[(2,2,2-trifluoroethyl)sulphinyl]phenyl}-3-(trifluoromethyl)-1H-1,2,4-triazole-5-amine (known from WO2006/043635) (CAS 885026-50-6), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457) (CAS 637360-23-7), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6) , 4-(but-2-yn-1-yloxy)-6-(3,5-dimethylpiperidin-1-yl)-5-fluoropyrimidine (known from WO2004/099160) (CAS 792914-58-0), PF1364 (known from JP2010/018586) (CAS 1204776-60-2), N-[(2E)-1-[(6-chloropyridin-3-yl)methyl]pyridin-2(1H)-ylidene]-2,2,2-trifluoroacetamide (known from WO2012/029672) (CAS 1363400-41-2), (3E)-3-[1-[(6-chloro-3-pyridyl)methyl]-2-pyridylidene]-1,1,1-trifluoro-propan-2-one (known from WO2013/144213) (CAS 1461743-15-6), ,N-[3-(benzylcarbamoyl)-4-chlorophenyl]-1-methyl-3-(pentafluoroethyl)-4-(trifluoromethyl)-1H-pyrazole-5-carboxamide (known from WO2010/051926) (CAS 1226889-14-0), 5-bromo-4-chloro-N-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-N-(cis-1-oxido-3-thietanyl)-benzamide, 4-[5-(3,5-dichlorophenyl)-4,5-dihydro-5-(trifluoromethyl)-3-isoxazolyl]-2-methyl-N-(trans-1-oxido-3-thietanyl)-benzamide and 4-[(5S)-5-(3,5-dichlorophenyl)-4,5-dihydro-5-(trifluoromethyl)-3-isoxazolyl]-2-methyl-N-(cis-1-oxido-3-thietanyl) benzamide (known from WO 2013/050317 A1) (CAS 1332628-83-7), N-[3-chloro-1-(3-pyridinyl)-1H-pyrazol-4-yl]-N-ethyl-3-[(3,3,3-trifluoropropyl)sulfinyl]-propanamide, (+)-N-[3-chloro-1-(3-pyridinyl)-1H-pyrazol-4-yl]-N-ethyl-3-[(3,3,3-trifluoropropyl)sulfinyl]-propanamide and (-)-N-[3-chloro-1-(3-pyridinyl)-1H-pyrazol-4-yl]-N-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2E)-3-chloro-2-propen-1-yl amino] -1-[2,6-dichloro-4-(trifluoromethyl)phenyl]-4-(trifluoromethyl)sulfinyl]-1H-pyrazole-3-carbonitrile (known from CN 101337937 A) (CAS 1105672-77-2), 3-bromo-N-[4-chloro-2-methyl-6-[(methylamino)thioxomethyl]phenyl]-1-(3-chloro-2-pyridinyl)-1H-pyrazole-5-carboxamide, (Liudaibenjiaxuanan, known from CN 103109816 A) (CAS 1232543-85-9); N-[4-chloro-2-[[(1,1-dimethylethyl)amino]carbonyl]-6-methylphenyl]-1-(3-chloro-2-pyridinyl)-3-(fluoromethoxy)-1H-Pyrazole-5-carboxamide (known from WO 2012/034403 A1) (CAS 1268277-22-0), N-[2-(5-amino-1,3,4-thiadiazol-2-yl)-4-chloro-6-methylphenyl]-3-bromo-1-(3-chloro-2-pyridinyl)-1H-pyrazole-5-carboxamide (known from WO 2011/085575 A1) (CAS 1233882-22-8), 4-[3-[2,6-dichloro-4-[(3,3-dichloro-2-propen-1-yl)oxy]phenoxy]propoxy]-2-methoxy-6-(trifluoromethyl)-pyrimidine (known from CN 101337940 A) (CAS 1108184-52-6); (2E)- and 2(Z)-2-[2-(4-cyanophenyl)-1-[3-(trifluoromethyl) phenyl]ethylidene]-N-[4-(difluoromethoxy)phenyl]-hydrazinecarboxamide (known from CN 101715774 A) (CAS 1232543-85-9); 3-(2,2-dichloroethenyl)-2,2-dimethyl-4-(1H-benzimidazol-2-yl)phenyl-cyclopropanecarboxylic acid ester (known from CN 103524422 A) (CAS 1542271-46-4); (4aS)-7-chloro-2,5-dihydro-2-[[(methoxycarbonyl)[4-[(trifluoromethyl)thio]phenyl]amino]carbonyl]-indeno[1,2-e][1,3,4]oxadiazine-4a(3H)-carboxylic acid methyl ester (known from CN 102391261 A) (CAS 1370358-69-2); 6-deoxy-3-O-ethyl-2,4-di-O-methyl-, 1-[N-[4-[1-[4-(1,1,2,2,2-pentafluoroethoxy) phenyl]-1H-1,2,4-triazol-3-yl]phenyl]carbamate]-α-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1]octane (CAS 1253850-56-4), (8-anti)-8-(2-cyclopropylmethoxy-4-trifluoromethyl-phenoxy)-3-(6-trifluoromethyl-pyridazin-3-yl)-3-aza-bicyclo[3.2.1 ]octane (CAS 933798-27-7), (8-syn)-8-(2-cyclopropylmethoxy-4-trifluoromethyl-phenoxy)-3-(6-trifluoromethyl-pyridazin-3-yl)-3-aza-bicyclo[3.2.1 ]octane (known from WO 2007040280 A1, WO 2007040282 A1) (CAS 934001-66-8) and N-[3-chloro-1-(3-pyridinyl)-1H-pyrazol-4-yl]-N-ethyl-3-[(3,3,3-trifluoropropyl)thio]-propanamide (known from WO 2015/058021 A1, WO 2015/058028 A1) (CAS 1477919-27-9).

The Insecticides and combinations thereof may be used in a curative or preventive manner.

Insecticides especially used in the context of the present invention are selected from the group I-2 consisting of:
Abamectin, A1 Phosphide, Azadirachtin, *Bacillus firmus* strain I-1582, Cadusafos, Carbofuran, Chloropicrin, Dichloropropene, Dimethyl-sulfid, Clothianidin, Spirotetramat, Flupyradifurone, Tetraniliprole, *Bacillus firmus* GB126, Imidacloprid, Flubendiamide, Deltamethrin, Thiacloprid, Spiromesifen, Ethiprole, Ethoprophos, *Paecilomyces lilacinus* 251, Terpenoid blend (e.g., natural extracts or simulated blend of *Chenopodium ambrosioides* (product known as Requiem®, which contains a mixture of three terpenes, i.e. α-terpinene (around 10%), p-cymene (around 3.75%) and limonene (around 3%), Fenamiphos, *Flavobacterium sp* strain H492, Fipronil, Fluazaindolizine, Fluensulfone, Fluopyram, Fosthiazate, Beta-Cyfluthrin, Thiodicarb, Triflumuron, Methiocarb, Cyfluthrin, Spirodiclofen,Silafluofen, Fenthion, Imicyafos, Iprodione, Metam-Sodium, Methylbromide, *Myrothecium verrucaria* in particular strain AARC-0255, Oxamyl, Sulphuryl fluoride, Tioxazafen, Inclosamide, Tralomethrin, Triafamone, Carbendazim, Carboxin, Difenoconazole, Ethoxysulfuron, Fenamidon, Fenoxaprop-P-Ethyl, Fluopicolide, Fluoxastrobin, Fluquinconazole, Fosetyl-Al, Prochloraz, Propineb, Prothioconazole, Spirotetramat, Tebuconazole, Tembotrione, Thiacloprid, Thiodicarb, Thiram, Triadimenol, Trifloxystrobin, Triflumuron, Cyflumetofen, Afidopyropen, Broflanilide, Sulfoxaflor, Cyazypyr, Rynaxypyr, Fluazaindolizine, Triflumezopyrim and Tioxazafen.

In one preferred embodiment, Insecticides are selected from the group I-3 consisting of:
Clothianidin, Spirotetramat, Flupyradifurone, Tetraniliprole, *Bacillus firmus* GB126, Imidacloprid, Flubendiamide, Deltamethrin, Thiacloprid, Spiromesifen, Ethiprole, *Paecilomyces lilacinus* 251, Terpenoid blend (e.g. Requiem®), Fipronil, Beta-Cyfluthrin, Thiodicarb, Triflumuron, Methiocarb, Cyfluthrin, Cyflumetofen, Afidopyropen, Broflanilide, Sulfoxaflor, Cyazypyr, Rynaxypyr, Fluazaindolizine, Triflumezopyrim and Tioxazafen.

In a more preferred embodiment, Insecticides are selected from the group I-4 consisting of:
Clothianidin, Spirotetramat, Flupyradifurone, Tetraniliprole, *Bacillus firmus* GB126, Imidacloprid, Flubendiamide, Deltamethrin, Thiacloprid, Spiromesifen, Ethiprole, *Paecilomyces lilacinus* 251, Terpenoid blend (e.g. Requiem®) and Fipronil.

In another more preferred embodiment, Insecticides are selected from the group I-5 consisting of:
Cyflumetofen, Afidopyropen, Broflanilide, Sulfoxaflor, Cyazypyr, Rynaxypyr, Fluazaindolizine, Triflumezopyrim and Tioxazafen.

In an even more preferred embodiment, Insecticides are selected from the group I-6 consisting of:
Clothianidin, Spirotetramat, Flupyradifurone, Tetraniliprole and *Bacillus firmus* GB126.

### Fungicides

The active ingredients specified herein by their Common Name are known and described, for example, in The Pesticide Manual (16th Ed.British Crop Protection Council) or can be searched in the internet (e.g. www.alanwood.net/pesticides).

In one embodiment the fungicidal active ingredient are selected from the group F-1 consisting of fifteen subgroups being
1) Inhibitors of the ergosterol biosynthesis, for example (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) Pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1-({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) 2-[4-(4-chlorophenoxy)-2-(trifluoromethyl)phenyl]-1-(1H-1,2,4-triazol-1-yl)propan-2-ol, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{ [3-(2,2,3,3-tetrafluoropropoxy)phenyl]sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxy]-2-methylpyridin-3-yl}-N-ethyl-N-methylimidoformamide, (1.078) N'-{5-bromo-6-[(cis-4-isopropylcyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) Mefentrifluconazole, (1.082) Ipfentrifluconazole;
2) Inhibitors of the respiratory chain at complex I or II, for example (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-c arboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3S)-1,1,3-trimethyl-2,3-dihydro-1H-inden-4-yl]-1H-pyrazole-4-carboxamide, (2.030) 3-(difluoromethyl)-N-(7-fluoro-1,1,3-trimethyl-2,3-dihydro-1H-inden-4-yl)-1-methyl-1H-pyrazole-4-carboxamide, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{ [4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(lR,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide;
3) Inhibitors of the respiratory chain at complex III, for example (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate;
4) Inhibitors of the mitosis and cell division, for example (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine;
5) Compounds capable to have a multisite action, for example (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile;
6) Compounds capable to induce a host defence, for example (6.001) acibenzolar-S-methyl, (6.002) isotianil, (6.003) probenazole, (6.004) tiadinil;
7) Inhibitors of the amino acid and/or protein biosynthesis, for example (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinolone;
8) Inhibitors of the ATP production, for example (8.001) silthiofam;
9) Inhibitors of the cell wall synthesis, for example (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one;
10) Inhibitors of the lipid and membrane synthesis, for example (10.001) propamocarb, (10.002) propamocarb hydrochloride, (10.003) tolclofos-methyl;
11) Inhibitors of the melanin biosynthesis, for example (11.001) tricyclazole, (11.002) 2,2,2-trifluoroethyl {3-methyl-1-[(4-methylbenzoyl)amino]butan-2-yl}carbamate;
12) Inhibitors of the nucleic acid synthesis, for example (12.001) benalaxyl, (12.002) benalaxyl-M (kiralaxyl), (12.003) metalaxyl, (12.004) metalaxyl-M (mefenoxam);
13) Inhibitors of the signal transduction, for example (13.001) fludioxonil, (13.002) iprodione, (13.003) procymidone, (13.004) proquinazid, (13.005) quinoxyfen, (13.006) vinclozolin;
14) Compounds capable to act as an uncoupler, for example (14.001) fluazinam, (14.002) meptyldinocap;
15) Further compounds, for example (15.001) Abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) Oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) 2,6-dimethyl-1H,5H-[1,4]dithiino[2,3-c:5,6-c']dipyrrole-1,3,5,7(2H,6H)-tetrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl -1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) 2-{2-[(7,8-difluoro-2-methylquinolin-3-yl)oxy]-6-fluorophenyl}propan-2-ol, (15.042) 2-{2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) 3-(4,4-difluoro-3,3-dimethyl-3,4-dihydroisoquinolin-1-yl)quinoline, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one.

The fungicides and the mixtures of fungicides mentioned herein may be used in a curative or preventive manner.

Fungicides used in the context of the present invention are selected from the group F-2 consisting of:
(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 2,6-dimethyl-1H,5H-[1,4]dithiino[2,3-c:5,6-c']dipyrrole-1,3,5,7(2H,6H)-tetrone, 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, 2-fluoro-6-(trifluoromethyl)-N-(1,1,3-trimethyl-2,3-dihydro-1H-inden-4-yl)benzamide, 3-(difluoromethyl)-1-methyl-N-(1,1,3-trimethyl-2,3-dihydro-1H-inden-4-yl)-1H-pyrazole-4-carboxamide, 3-(difluoromethyl)-N-(7-fluoro-1,1,3-trimethyl-2,3-dihydro-1H-inden-4-yl)-1-methyl-1H-pyrazole-4-carboxamide, 3-chloro-4-(2,6-difluorophenyl)-6-methyl-5-phenylpyridazine, 4-(2-bromo-4-fluorophenyl)-N-(2-chloro-6-fluorophenyl)-1,3-dimethyl-1H-pyrazol-5-amine , 5-fluoro-2-[(4-methylbenzyl)oxy]pyrimidin-4-amine, Ametoctradin, Amisulbrom, Azoxystrobin, Benthiavalicarb, Benzovindiflupyr, Bitertanol, Bixafen, Boscalid, Captan, Carbendazim, Carboxin, Carpropamid, Chlormequat-chloride, Chlorothalonil, Copper fungicides, in particular copper oxychloride, copper sulfate, Cyazofamid, Cyflufenamid, Cymoxanil, Cyproconazole, Cyprodinil, Diclocymet, Difenoconazole, Dimethomorph, Dimoxystrobin, Diniconazole, Dithianon, Edifenphos, Epoxiconazole, Famoxadone, Fenamidone, Fenhexamid, Fenpropidin, Fenpropimorph, Fenpyrazamine, Ferimzone, Fluazinam, Fludioxonil, Fluindapyr, Fluopicolide, Fluopyram, Fluoxastrobin, Fluquinconazole, Flusilazole, Fluthianil, Flutriafol, Fluxapyroxad, Folpet, Fosetyl or salts and esters thereof, in particular Fosetyl-aluminium, Furametpyr, Gentamycin, Hexaconazole, Hymexazol, Imazalil, Ipconazole, Ipfentrifluconazole, Iprobenfos (IBP), Iprodione, Iprovalicarb, Isofetamid, Isoprothiolane, Isopyrazam, Isotianil, Kasugamycin, Kresoxim-methyl, Lyserphenvalpyr, Mancozeb, Mandestrobin, Mandipropamid, Maneb, Mefenoxam, Mefentrifluconazole, Mepiquat-chloride, Meptyldinocap, Metalaxyl, Mefenoxam, Metconazole, Metiram, Metominostrobin, Metrafenone, Myclobutanil, N-(5-chloro-2-isopropylbenzyl)-N-cyclopropyl-3-(difluoromethyl)-5-fluoro-1-methyl-1H-pyrazole-4-carboxamide , Orysastrobin, Oxathiapiprolin, Paclobutrazole, Penconazole, Pencycuron, Penflufen, Penthiopyrad, Phosphonic acid (H3PO3), Phthalide, Picarbutrazox, Picoxystrobin, Probenazole, Prochloraz, Propamocarb, Propiconazole, Propineb, Proquinazid, Prothioconazole, Pydiflumetofen, Pyraclostrobin, Pyraziflumid, Pyrimethanil, Pyriofenone, Pyroquilon, Quinofumelin , Quinoxyfen, Quinoxyfen, Quintozene, Saponin, Sedaxane, Silthiofam, Simconazole, Spiroxamine, Streptomycin, Sulphur, Tebuconazole, Tebufloquin, Tetraconazole, Thiabendazole, Thifluzamide, Thiophanate-methyl, Thiram, Tiadinil, Tolprocarb, Triadimefon, Triadimenol, Tricyclazole, Trifloxystrobin, Triticonazole, Validamycin, Ziram and the esters and/or agronomically acceptable salts of these fungicides.

Preferred Fungicides are selected from the group F-3 consisting of:
(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, 2,6-dimethyl-1H,5H-[1,4]dithiino[2,3-c:5,6-c']dipyrrole-1,3,5,7(2H,6H)-tetrone, 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, 2-fluoro-6-(trifluoromethyl)-N-(1,1,3-trimethyl-2,3-dihydro-1H-inden-4-yl)benzamide, 3-(difluoromethyl)-1-methyl-N-(1,1,3-trimethyl-2,3-dihydro-1H-inden-4-yl)-1H-pyrazole-4-carboxamide, 3-(difluoromethyl)-N-(7-fluoro-1,1,3-trimethyl-2,3-dihydro-1H-inden-4-yl)-1-methyl-1H-pyrazole-4-carboxamide, 3-chloro-4-(2,6-difluorophenyl)-6-methyl-5-phenylpyridazine, 4-(2-bromo-4-fluorophenyl)-N-(2-chloro-6-fluorophenyl)-1,3-dimethyl-1H-pyrazol-5-amine , 5-fluoro-2-[(4-methylbenzyl)oxy]pyrimidin-4-amine, Ametoctradin, Amisulbrom, Azoxystrobin, Benzovindiflupyr, Bitertanol, Bixafen, Boscalid, Carpropamid, Chlorothalonil, Cyproconazole, Difenoconazole, Epoxiconazole, Famoxadone, Fenamidone, Fenhexamid, Fludioxonil, Fluindapyr, Fluopicolide, Fluopyram, Fluoxastrobin, Fluquinconazole, Fluxapyroxad, Fosetyl or salts and esters thereof, in particular Fosetyl-aluminium, Ipconazole, Ipfentrifluconazole, Iprodione, Iprovalicarb, Isofetamid, Isoprothiolane, Isopyrazam, Isotianil, Kresoxim-methyl, Lyserphenvalpyr , Mandestrobin, Mandipropamid, Mefenoxam, Mefentrifluconazole, Metalaxyl, Mefenoxam, Metconazole, Metominostrobin, N-(5-chloro-2-isopropylbenzyl)-N-cyclopropyl-3-(difluoromethyl)-5-fluoro-1-methyl-1H-pyrazole-4-carboxamide , Oxathiapiprolin, Pencycuron, Picarbutrazox, Picoxystrobin, Propamocarb, Propiconazole, Propineb, Prothioconazole, Pydiflumetofen, Pyraclostrobin, Pyraziflumid, Pyrimethanil, Pyriofenone, Quinofumelin , Quinoxyfen, Sedaxane, Silthiofam, Spiroxamine, Tebuconazole, Tetraconazole, Tolprocarb, Triadimefon, Triadimenol, Trifloxystrobin, and the esters and/or agronomically acceptable salts of these fungicides.

Preferred mixtures of fungicides used in the context of the present invention are selected from the group F4 consisting of :
mixtures of (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate and other fungicides mentioned herein, mixtures of 2,6-dimethyl-1H,5H-[1,4]dithiino[2,3-c:5,6-c']dipyrrole-1,3,5,7(2H,6H)-tetrone, esters and/or salts thereof and other fungicides mentioned herein, mixtures of 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate and other fungicides mentioned herein, mixtures of 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone and other fungicides mentioned herein, mixtures of Ametoctradin and other fungicides mentioned herein, mixtures of Bitertenol and other fungicides mentioned herein, mixtures of Bixafen and/or salts thereof and other fungicides mentioned herein, mixtures of Carpropamid and other fungicides mentioned herein, mixtures of Cyproconazole and other fungicides mentioned herein, mixtures of Epoxiconazole and other fungicides mentioned herein, mixtures of Fenhexamid and other fungicides mentioned herein, mixtures of Fenamidone and other fungicides mentioned herein, mixtures of Fluopyram and other fungicides mentioned herein, mixtures of Fluopicolide and other fungicides mentioned herein, mixtures of Fluoxastrobin and other fungicides mentioned herein, mixtures of Fluxapyroxad and other fungicides mentioned herein,mixtures of Fluquinconazole and other fungicides mentioned herein, mixtures of Fosetyl or salts and esters thereof, in particular Fosetyl-aluminium and other fungicides mentioned herein, mixtures of Ipfentrifluconazole and other fungicides mentioned herein, mixtures of Iprovalicarb, and other fungicides mentioned herein, mixtures of Isotianil and other fungicides mentioned herein, mixtures of Lyserphenvalpyr and other fungicides mentioned herein, mixtures of Mefentrifluconazole and other fungicides mentioned herein, mixtures of Metalaxyl and other fungicides mentioned herein, mixtures of Mefenoxam and other fungicides mentioned herein, mixtures of Metominostrobin and other fungicides mentioned herein, mixtures of N-(5-chloro-2-isopropylbenzyl)-N-cyclopropyl-3-(difluoromethyl)-5-fluoro-1-methyl-1H-pyrazole-4-carboxamide and other fungicides mentioned herein, mixtures of Pencycuron and other fungicides mentioned herein, mixtures of Penflufen and other fungicides mentioned herein, mixtures of Propamocarb and other fungicides mentioned herein, mixtures of Propineb and other fungicides mentioned herein, mixtures of Pyrimethanil and other fungicides mentioned herein, mixtures of Pyraclostrobin and other fungicides mentioned herein, mixtures of Prothioconazole and other fungicides mentioned herein, mixtures of Spiroxamine and other fungicides mentioned herein, mixtures of Tebuconazole and other fungicides mentioned herein, mixtures of Triadimenol and other fungicides mentioned herein, mixtures of Triadimefon and other fungicides mentioned herein, mixtures of Trifloxystrobin and other fungicides mentioned herein.

Particularly preferred mixtures of fungicides used in the context of the present invention are selected from the group F-5 consisting of :
mixtures of Prothioconazole and Fluopyram, mixtures of Prothioconazole and Tebuconazole, mixtures of Prothioconazole, Bixafen, and Tebuconazole,
mixtures of Prothioconazole, Bixafen, and Trifloxystrobin, mixtures of Prothioconazole, Bixafen, and Spiroxamine, mixtures of Prothioconazole, Bixafen, and Chlorothalonil, mixtures of Prothioconazole and Tebuconazole and Metalaxyl, mixtures of Prothioconazole, Bixafen, and Fluopyram, mixtures of Prothioconazole and Fluoxastrobin, mixtures of Prothioconazole and Trifloxystrobin, mixtures of Prothioconazole and Pencycuron, mixtures of Prothioconazole and Spiroxamine, mixtures of Tebuconazole and Bixafen, mixtures of Tebuconazole and Metalaxyl, mixtures of Tebuconazole and Fluoxastrobin, mixtures of Tebuconazole and Trifloxystrobin, mixtures of Tebuconazole and Fenhexamid, mixtures of Tebuconazole and Fluopicolide, mixtures of Tebuconazole and Spiroxamine, mixtures of Tebuconazole and Pencycuron, mixtures of Fluopyram and Tebuconazole, mixtures of Fluopyram and Spiroxamine, mixtures of Fluopyram and Pyrimethanil, mixtures of Fluopyram and Trifloxystrobin, mixtures of Fluopyram and Triadimenol, mixtures of Fluopyram and Fosetyl or salts and esters thereof, in particular Fosetyl-aluminium, mixtures of Fluopyram and Imidacloprid, mixtures of Fluopyram and Spiroxamine, mixtures of Fluopyram and Fluoxastrobin, mixtures of Bixafen and Spiroxamine, mixtures of Bixafen and Trifloxystrobin, mixtures of Bixafen and Chlorothalonil, mixtures of Bixafen and Fluoxastrobin, mixtures of Trifloxystrobin and Isotianil, mixtures of Trifloxystrobin and Metalaxyl,
mixtures of Trifloxystrobin and Propineb, mixtures of Trifloxystrobin and Pyrimethanil, mixtures of Trifloxystrobin and Fosetyl or salts and esters thereof, in particular Fosetyl-aluminium,
mixtures of Trifloxystrobin and Fluopicolide, mixtures of Propineb and Fluopicolide, mixtures of Fosetyl or salts and esters thereof, in particular Fosetyl-aluminium,
and Fluopicolide, mixtures of Bixafen and Ipfentrifluconazole, mixtures of Bixafen and Mefentrifluconazole,
mixtures of Metalaxyl and Ipfentrifluconazole, mixtures of Metalaxyl and Mefentrifluconazole, mixtures of Prothioconazole and Ipfentrifluconazole, mixtures of Prothioconazole and Mefentrifluconazole, mixtures of Tebuconazole and Ipfentrifluconazole, mixtures of Tebuconazole and Mefentrifluconazole, mixtures of Trifloxystrobin and Ipfentrifluconazole, mixtures of Trifloxystrobin and Mefentrifluconazole, mixtures of Fluoxastrobin and Ipfentrifluconazole, mixtures of Fluoxastrobin and Mefentrifluconazole, mixtures of Fluopyram and Ipfentrifluconazole, mixtures of Fluopyram and Mefentrifluconazole, mixtures of Spiroxamine and Ipfentrifluconazole, mixtures of Spiroxamine and Mefentrifluconazole, mixtures of Pydiflumetofen and Ipfentrifluconazole, mixtures of Pydiflumetofen and Mefentrifluconazole, mixtures of Pyraclostrobin and Ipfentrifluconazole, mixtures of Pyraclostrobin and Mefentrifluconazole, mixtures of Fluxapyroxad and Ipfentrifluconazole, mixtures of Fluxapyroxad and Mefentrifluconazole.

### SeedGrowth

Seeds of plants comprising the events and alleles as disclosed below in table A, B and C can also be treated with seed treatment.

Seeds can be coated with a coating comprising a compound selected from the following fungicides, insecticides, safeners and/or biologics.

Preferred fungicides for seed treatment of seeds of plants comprising the events and alleles as disclosed below in table A, B and C are selected from the group consisting of fungicides, insecticides, biologics as disclosed below.

Preferred fungicides for seed treatment of seeds are selected from the group SG-1 consisting of

Benzovindiflupyr, Carbendazim, Carboxin, Difenoconazole, Ethaboxam, Fludioxonil, Fluquinconazole, Fluxapyroxad, Ipconazole, Ipfentrifluconazole,Isotianil, Mefenoxam, Mefentrifluconazole, Metalaxyl, Pencycuron, Penflufen, Penthiopyrad, Prothioconazole, Prochloraz, Pyraclostrobin, Sedaxane, Silthiofam, Tebuconazole, Trifloxystrobin, Triticonazole, Ethaboxam (SCC), Penthiopyrad (DPX pipeline), Benzovindiflupyr (SYN pipeline), Bixafen, (see biologicals), Dimethomorph, Fenamidone, Fluopicolide, Fluoxastrobin, Flutolanil, Tolclophos-methyl, Azoxystrobin, Chlorothalonil, Cyproconazole, Cyprodinil, Diniconazole, Fluopyram, Flutriafol, Fluxapyroxad, Imazalil, Isopyrazam, Isotianil, Iprodione, Metconazole, Myclobutanil, Picoxystrobin, Pyrimethanil, Tetraconazole, Thiabendazole, Thiophanate-methyl, Triadimenol, Thiram, Triflumizole, Ziram, 2,6-dimethyl-1H,5H-[1,4]dithiino[2,3-c:5,6-c']dipyrrole-1,3,5,7(2H,6H)-tetrone, 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate (Thiazolylpiperidin , N-(5-chloro-2-isopropylbenzyl)-N-cyclopropyl-3-(difluoromethyl)-5-fluoro-1-methyl-1H-pyrazole-4-carboxamide Cypropamide, Picarbutrazox and Oxathiapiprolin.

Preferred insecticides for seed treatment of seeds are selected from the group SG-2 consisting of

Abamectin, Afidopyropen, Bifenthrin, Carbofuran, Carbendazim, Carboxin, Clothianidin, Cyazypyr, Cypermethrin, Deltamethrin, Difenoconazole, Ethoxysulfuron, Fenamidon, Fenoxaprop-P-Ethyl, Ethiprole, Fipronil, Fluazaindolizine, Flupyradifurone, Flubendiamide, Fluopicolide, Fluopyram, Fluoxastrobin, Fluquinconazole, Fosetyl-Al, Imidacloprid, Prochloraz, Propineb, Prothioconazole, Lambda-Cyhalothrin, Methiocarb, Rynaxypyr, Spirotetramat, Spinosad, Tebuconazole, Tembotrione, Thiacloprid, Thiram, Thiametoxam, Thiodicarb, Thioxazafen, (MON pipeline Nematicide), Fluazaindolizine (DPX pipeline Nematicide), Lambda-Cyhalothrin (SYN), Bifenthrin (FMC), Cypermethrin (few companies), Acetamiprid, ß-Cyfluthrin, Flubendiamide, Thiacloprid, Chlorphyriphos, Metamidophos, Phorate, Sulfoxaflor, Tefluthrin, Triflumuron, Tetraniliprole, Triadimenol, Trifloxystrobin, Triflumuron, Broflanilide (MCI 8007 - Mitsui/BASF), Cycloxaprid and Spinetoram.

Preferred safeners for seed treatment of seeds are selected from the group SG-3 consisting of Cyprosulfamide, Isoxadifen, Isoxadifen-ethyl, Lipo-Chito-Oligosaccharide (LCO), Mefenpyr and Mefenpyr-diethyl.

Preferred Biologicals and biological active ingredients for seed treatment of seeds are selected from the group SG-4 consisting of
*Bacilus firmus,* especially *Bacillus firmus* GB126, Bacillus subtilis, *Bacillus pumilus especially Bacillus pumilus* QST 2808, *Bacillus pumilus* GB34, *Pasteuria usage* strains (e.g. as used in ECONEM), *Rhizobium* spp, especially Rhizobium *tropici* SP25, *Pseudomonas fluorescens, Pseudomonas chloropsis, Penicilium bilaii, Rhizobium japonicum, Purnate Varroacide, Chenopodioum quinoa saponins*, a recombinant exosporium-producing *Bacillus thuiringienses* cell (especially *B. thuringiensis* BT013A) that expresses a fusion protein comprising:
   1) an endoglucanase, (especially from *Bacillus subtilis*) having SEQ ID NO. 2 and
   2) a targeting sequence having at least about 43% identity with amino acids 20-35 of SEQ ID NO: 1, wherein the identity with amino acids 25-35 is at least about 54%,
   and a recombinant exosporium-producing *Bacillus thuringiensis* cell that expresses a fusion protein comprising:
   1) a phosphoplipase having SEQ ID NO. 3 and
   2) a targeting sequence having at least about 43% identity with amino acids 20-35 of SEQ ID NO: 1, wherein the identity with amino acids 25-35 is at least about 54%.

More preferred Biologicals and biological active ingredients for seed treatment of seeds are selected from the group SG-5 consisting of:
*Bacilus firmus,* especially *Bacillus firmus* GB126, Bacillus subtilis, *Bacillus pumilus especially Bacillus pumilus* QST 2808, *Bacillus pumilus* GB34, a recombinant exosporium-producing *Bacillus thuiringienses* cell (especially *B. thuringiensis* BT013A) that expresses a fusion protein comprising:
   1) an endoglucanase, (especially from *Bacillus subtilis*) having SEQ ID NO. 2 and
   2) a targeting sequence having at least about 43% identity with amino acids 20-35 of SEQ ID NO: 1, wherein the identity with amino acids 25-35 is at least about 54%,
   and a recombinant exosporium-producing *Bacillus thuringiensis* cell that expresses a fusion protein comprising:
   1) a phosphoplipase having SEQ ID NO. 3 and
   2) a targeting sequence having at least about 43% identity with amino acids 20-35 of SEQ ID NO: 1, wherein the identity with amino acids 25-35 is at least about 54%.

In another more preferred embodiment, preferred active ingredients for seed treatment of seeds are selected from the group SG-6 consisting of:
ß-Cyfluthrin, *Bradyrhizobium japonicum*, Carbendazim, Carboxin Clothianidin, Cypermethrin, Deltamethrin, Difenoconazole, Ethoxysulfuron, Fenamidon, Fenoxaprop-P-Ethyl, Flubendiamide, Fluopicolide, Fluopyram, Fluoxastrobin, Fluquinconazole, Fosetyl-Al, Fipronil, Prochloraz, Propineb, Prothioconazole, *Pseudomonas Fluorescens*, Spirotetramat, Tebuconazole, Tembotrione, Thiacloprid, Thiodicarb, Thiram, Triadimenol, Trifloxystrobin and Triflumuron.

Particularly preferred combinations of active ingredients for seed treatment used in the context of the present invention are selected from the group consisting of SG-7:
combination of Clothianidin and *B. firmus* GB126,
combination of Imidacloprid and Thiodicarb,
combination of Imidacloprid and Prothioconazole,
combination of Clothianidin and Carboxin and Metalaxyl and Trifloxystrobin,
combination of Metalaxyl and Prothioconazole and Tebuconazole,
combination of Clothianidin and beta-Cyfluthrin,
combination of Prothioconazole and Tebuconazole,
combination of Clothianidin and Imidacloprid and Prothioconazole and Tebuconazole,
combination of Carbendazim and Thiram,
combination of Imidacloprid and Methiocarb,
combination of Metalaxyl and Penflufen and Prothioconazole,
combination of Imidacloprid Prothioconazole,
combination of Imidacloprid Tefluthrin,
combination of Imidacloprid Pencycuron,
combination of Imidacloprid Penflufen,
combination of Fluoxastrobin and Prothioconazole and Tebuconazole,
combination of Metalaxyl and Trifloxystrobin ,
combination of Penflufen and Trifloxystrobin,
combination of Prothioconazole and Tebuconazole,
combination of Fluoxastrobin and Prothioconazole and Tebuconazole and Triazoxide,
combination of Imidacloprid and Methiocarb and Thiram,
combination of Clothianidin and beta-Cyfluthrin,
combination of Clothianidin and Fluoxastrobin and Prothioconazole and Tebuconazole,
combination of Fluopyram and Fluoxastrobin and Triadimenol,
combination of Metalaxyl and Trifloxystrobin,
combination of Imidacloprid and Ipconacole,
combination of Difenoconazol and Fludioxonil and Tebuconazole,
combination of Imidacloprid + Tebuconazole,
combination of Imidacloprid and Prothioconazole and Tebuconazole and
combination of Metalaxyl and Prothioconazole and Tebuconazole.

In one preferred embodiment, a combination of active ingredients for seed treatment is selected from the group SG-8 consisting of:
combination of Clothianidin and *B. firmus* GB126,
combination of Imidacloprid and Thiodicarb,
combination of Imidacloprid and Prothioconazole,
combination of Clothianidin Carboxin, Metalaxyl, Trifloxystrobin,
combination of Metalaxyl, Prothioconazole Tebuconazole and
combination of Clothianidin and beta-Cyfluthrin and
combination of Prothioconazole and Tebuconazole.

In the context of the present invention, "increasing yield" means a significant increase in yield by compared with the untreated plant, preferably a significant increase by at least 1% such as by 1% to 3%, compared with the untreated plant (100% yield), i.e. the yield of the treated plants is at least 101% compared to the yield (100%) of the untreated plant; more preferably, the yield is even more increased ba at least 2%, more preferably at least 5% such as by 2% to 5% (yield from 102% to 105%), by 2 to 10 % (yield from 102% to 110%) or by 5% to 20% (yield of from105% to 120 %). The yield increase may be achieved by curative treatment, i.e. for treatment of already infected plants, or by protective treatment, for protection of plants which have not yet been infected.

In the context of the present invention, "to treat with" means to contact a plant or part of a plant with an effective amount of an active ingredient (AI) or a combination thereof or to coat a seed with an active AI or a combination thereof.

"Active ingredient" shall refer to compounds used in agriculture. Agriculture encompasses the production of food and feed crops, forestry, the protection of stored products including food, feed but also other materials of plant origin. Preferably agriculture shall encompass the production of food and feed crops, forestry, the protection of stored products being food, feed, and materials of plant origin. Active ingredients according to the invention are not applied to humans or animals as a medical or therapeutic treatment.

"Insecticides" shall refer to active ingredients which are capable of controlling phytopathogenic pests including but not limited to insects, mites, nematodes.

In the context of the present invention the active ingredients according to the invention or to be used according to the invention may be a composition (i. e. a physical mixture) comprising at least one active ingredient. It may also be a combination of active ingredients composed from separate formulations of a single active ingredient component being active ingredient (tank-mix). Another example of a combination of active ingredients according to the invention is that the active ingredients are not present together in the same formulation, but packaged separately (combipack), i.e., not jointly preformulated. As such, combipacks include one or more separate containers such as vials, cans, bottles, pouches, bags or canisters, each container containing a separate component for an agrochemical composition, here at least one active ingredient. One example is a two-component combipack. Accordingly the present invention also relates to a two-component combipack, comprising a first component which in turn comprises an active ingredient, a liquid or solid carrier and, if appropriate, at least one surfactant and/or at least one customary auxiliary, and a second component which in turn comprises another active ingredient, a liquid or solid carrier and, if appropriate, at least one surfactant and/or at least one customary auxiliary. More details, e.g. as to suitable liquid and solid carriers, surfactants and customary auxiliaries are described below. A mixture or combination according to the invention shall mean/encompass a tank mix or a combipack.

In one embodiment an active ingredient or a combination of active ingredients as described above is used for increasing yield in Brassicaceae.

Thus, one embodiment also refers to a method to increase yield of a plant as described herein comprising the step of treating a plant or part of a plant as described herein with an effective amount of an active ingredient or a combination thereof

Especially, the following *Brassicaceae* comprising an event or allele as disclosed in tables A, B, and C below can be treated with an active ingredient or a combination of active ingredients as described in groups H-1, H-2, H-3, H-4, H-5, P-1, SAF-1, SAF-2, SAF-3, SAF-4, B-1, B-2, B-3, B-4, B-5, I-1, I-2, I-3,1-4,1-5,1-6, F-1, F-2, F-3, F-4, F-5, F-6, SG-1, SG-2, SG-3, SG-4, SG-5, SG-6, SG-7, SG-8.

In one embodiment, *Brassicaceae* plants suitable for treatments according to the invention are plants comprising the events or alleles listed in Table A. The events are characterized by reference to patent applications (column PA - incorporated by reference) or regulatory files (column RF -incorporated by reference) wherein they are described. Transgenic plants comprising the events can also be described by reference to the SEQ ID entries of the Sequence listings of the referenced patent applications (colum CS- herein incorporated by reference). These event characterizing sequences correspond to 5'flanking regions and junction sequences, 3' flanking regions and junction sequences, inserted transgene sequences, and/or complete sequences of the transgenic insert in the event. Transgenic plants suitable for treatments according to the invention comprise at least one, preferably all of the reference sequences.

Thus, in one embodiment, *Brassicaceae* plants suitable for treatments according to the invention are transgenic plants comprising at least one or more events characterized by the following OECD numbers: CGN-89111-8 or MON-88302-9 or MON-89249-2 or MON-00073-7 or ACS-BN003-6 or Brassicaceae plants comprising at least one or more the following alleles: POSH122, OGURA, R113, R40, R2000.

In another embodiment, *Brassicaceae* plants suitable for treatments according to the invention are transgenic *Brassicaceae* plants having at least one event or allele as present in reference seeds being deposited as: ATCC PTA-10955 or ATCC PTA-730 or ATCC PTA-730 or NCIMB41574 or ATCC PTA-850 or PTA-2485.

**Table A**

| **TYPE** | **NAME** | **ALTER NATIVE NAMES (List)** | **UNIQUE IDENTIFIER NUMBER** | **GENES** | **TYPE TRAIT** | **DEPOSIT NUMBER** | **PA** | **RF** | **CS** |
|---|---|---|---|---|---|---|---|---|---|
| EVENT1 | 23-18-17 | EVENT 18, pCGN3 828-212 / 86-18 | CGN-89111-8 | te, nptII | Modified oil/fatty acid | | | US94-090-01p | |
| EVENT2 | MON8 8302 | PV-BNHT2 672 | MON-88302-9 | cp4 epsps | Glyphosate tolerance | ATCC PTA-10955 | WO2011/153186 | US11-188-01p | SEQ ID Nos. 3, 4 , 5 and particularly SEQ ID No 6 |
| EVENT3 | RT200 | GT200 | MON-89249-2 | cp4 epsps, goxv247 | Glyphosate tolerance | | | US01-324-01p | |
| EVENT4 | RT73 | GT73, PV-BNGT0 4 | MON-00073-7 | cp4 epsps, goxv247 | Glyphosate tolerance | | WO2002/036831 | US98-216-01p | SEQ ID Nos 7 and 8 |
| EVENT5 | MS8 | | ACS-BN005-8 | bamase, bar | Male Sterility, Glufosinate Tolerance | ATCC PTA-730 | WO2001/041558 | US98-278-01p | SEQ ID Nos 13 and 18 |
| EVENT6 | RF3 | | ACS-BN003-6 | barstar, bar | Fertility Restoration, Glufosinate Tolerance | ATCC PTA-730 | WO2001/041558 | US98-278-01p | SEQ ID Nos 24 and 30 |
| ALLELE 1 | POSH1 22 | | not applicable | ind | Seed Shatter Resistance | NCIMB41 574 | WO2010/006732 | | SEQ ID No 27 |
| EVENT7 | MS11 | | | bar, barnase, barstar | Male Sterility, Glufosinate Tolerance | ATCC PTA-850 or PTA-2485 | WO2001/031042 | | SEQ ID Nos 8 and 10 |
| ALLELE 2 | Ogura | | not applicable | cms | Male Sterility | | | | |
| ALLELE 3 | R113 | | not applicable | Restorer | Fertility Restoration | | | | |
| ALLELE 4 | R40 | | not applicable | Restorer | Fertility Restoration | | | | |
| ALLELE 5 | R2000 | | not applicable | Restorer | Fertility Restoration | | | | |

In another embodiment, *Brassicaceae* plants suitable for treatments according to the invention are commercialized plants comprising the events or alleles listed in Table B. The events are characterized by reference to patent applications (column PA - incorporated by reference) or regulatory files (column RF -incorporated by reference) wherein they are described. Transgenic plants comprising the events can also be described by reference to the SEQ ID entries of the Sequence listings of the referenced patent applications (colum CS- herein incorporated by reference). These event characterizing sequences correspond to 5'flanking regions and junction sequences, 3' flanking regions and junction sequences, inserted transgene sequences, and/or complete sequences of the transgenic insert in the event. Transgenic plants suitable for treatments according to the invention comprise at least one, preferably all of the reference sequences.

Thus, in another embodiment, *Brassicaceae* plants suitable for treatments according to the invention are transgenic plants comprising at least one or more events characterized by the following OECD numbers: MON-88302-9 or MON-89249-2 or MON-00073-7 or ACS-BN003-6 or Brassicaceae plants comprising at least one or more the following alleles: POSH122, OGURA, R113, R40, R2000.

In another embodiment, *Brassicaceae* plants suitable for treatments according to the invention are transgenic *Brassicaceae* plants having at least one event or allele as present in reference seeds being deposited as: ATCC PTA-10955 or ATCC PTA-730 or ATCC PTA-730 or NCIMB41574 or ATCC PTA-850 or PTA-2485.

**Table B (commercialized plants)**

| **NAME** | **ALTER NATIVE NAMES (List)** | **UNIQUE IDENTIFIER NUMBER** | **GENES** | **TYPE TRAIT** | **DEPOSIT NUMBER** | **PA** | **RF** | **CS** |
|---|---|---|---|---|---|---|---|---|
| MON883 02 | PV-BNHT2 672 | MON-88302-9 | cp4 epsps | Glyphosate tolerance | ATCC PTA-10955 | WO2011/153186 | US11-188-01p | SEQ ID Nos. 3, 4 , 5 and particularly SEQ ID No 6 |
| RT200 | GT200 | MON-89249-2 | cp4 epsps, goxv247 | Glyphosate tolerance | | | US01-324-01p | |
| RT73 | GT73, PV-BNGT0 4 | MON-00073-7 | cp4 epsps, goxv247 | Glyphosate tolerance | | WO2002/036831 | US98-216-01p | SEQ ID Nos 7 and 8 |
| MS8 | | ACS-BN005-8 | bamase, bar | Male Sterility, Glufosinate Tolerance | ATCC PTA-730 | WO2001/041558 | US98-278-01p | SEQ ID Nos 13 and 18 |
| RF3 | | ACS-BN003-6 | barstar, bar | Fertility Restoration, Glufosinate Tolerance | ATCC PTA-730 | WO2001/041558 | US98-278-01p | SEQ ID Nos 24 and 30 |
| POSH 122 | | not applicable | not applicabl e | Seed Shatter Resistance | NCIMB41574 | WO2010/006732 | | SEQ ID No 27 |
| MS11 | | | bar, bamase, barstar | Male Sterility, Glufosinate Tolerance | ATCC PTA-850 or PTA-2485 | WO2001/031042 | | SEQ ID Nos 8 and 10 |
| Ogura | | not applicable | not applicabl e | Male Sterility | | | | |
| R113 | | not applicable | not applicabl e | Fertility Restoration | | | | |
| R40 | | not applicable | not applicabl e | Fertility Restoration | | | | |
| R2000 | | not applicable | not applicabl e | Fertility Restoration | | | | |

In yet another embodiment, *Brassicaceae* plants suitable for treatments according to the invention are plants comprising the recently developed events or alleles listed in Table C. The events are characterized by reference to patent applications (column PA - incorporated by reference) or regulatory files (column RF -incorporated by reference) wherein they are described. Transgenic plants comprising the events can also be described by reference to the SEQ ID entries of the Sequence listings of the referenced patent applications (colum CS- herein incorporated by reference). These event characterizing sequences correspond to 5'flanking regions and junction sequences, 3' flanking regions and junction sequences, inserted transgene sequences, and/or complete sequences of the transgenic insert in the event. Transgenic plants suitable for treatments according to the invention comprise at least one, preferably all of the reference sequences.

Thus, in another embodiment, *Brassicaceae* plants suitable for treatments according to the invention are transgenic plants comprising at least the event characterized by the following OECD numbers: MON-88302-9 or Brassicaceae plants comprising at least the following allele: POSH122.

In another embodiment, *Brassicaceae* plants suitable for treatments according to the invention are transgenic *Brassicaceae* plants having at least one event or allele as present in reference seeds being deposited as: ATCC PTA-10955 or NCIMB41574.

**Table C (Recently developed plants)**

| **TYPE** | **NAM E** | **ALTERNATIVE NAMES (List)** | **UNIQUE IDENTI FIER NUMBER** | **GENES** | **TYPE TRAIT** | **DEPOSIT NUMBER** | **PA** | **RF** | **CS** |
|---|---|---|---|---|---|---|---|---|---|
| EVENT | MON 88302 | PV-BNHT2672 | MON-88302-9 | cp4 epsps | Glyphosate tolerance | ATCC PTA-10955 | WO2011/ 153186 | US11-188-01p | SEQ ID Nos. 3, 4 , 5 and particularly SEQ ID No 6 |
| ALLELE | POSH 122 | | not applicabl e | not applicab le | Seed Shatter Resistance | NCIMB41574 | WO2010/ 006732 | | SEQ ID No 27 |

In one embodiment at least one active ingredient selected from group H-1 is used on a plant comprising EVENT1 or plant parts thereof, preferably for increasing yield.

In one embodiment at least one active ingredient selected from group H-2 is used on a plant comprising EVENT1 or plant parts thereof, preferably for increasing yield.

In one embodiment at least one active ingredient selected from group H-3 is used on a plant comprising EVENT1 or plant parts thereof, preferably for increasing yield.

In one embodiment at least one active ingredient selected from group H-4 is used on a plant comprising EVENT1 or plant parts thereof, preferably for increasing yield.

In one embodiment at least one active ingredient selected from group H-5 is used on a plant comprising EVENT1 or plant parts thereof, preferably for increasing yield.

In one embodiment at least one active ingredient selected from group P-1 is used on a plant comprising EVENT1 or plant parts thereof, preferably for increasing yield.

In one embodiment at least one active ingredient selected from group SAF-1 is used on a plant comprising EVENT1 or plant parts thereof, preferably for increasing yield.

In one embodiment at least one active ingredient selected from group SAF-2 is used on a plant comprising EVENT1 or plant parts thereof, preferably for increasing yield.

In one embodiment at least one active ingredient selected from group SAF-3 is used on a plant comprising EVENT1 or plant parts thereof, preferably for increasing yield.

In one embodiment at least one active ingredient selected from group SAF-4 is used on a plant comprising EVENT1 or plant parts thereof, preferably for increasing yield.

In one embodiment at least one active ingredient selected from group B-1 is used on a plant comprising EVENT1 or plant parts thereof, preferably for increasing yield.

In one embodiment at least one active ingredient selected from group B-2 is used on a plant comprising EVENT1 or plant parts thereof, preferably for increasing yield.

In one embodiment at least one active ingredient selected from group B-3 is used on a plant comprising EVENT1 or plant parts thereof, preferably for increasing yield.

In one embodiment at least one active ingredient selected from group B-4 is used on a plant comprising EVENT1 or plant parts thereof, preferably for increasing yield.

In one embodiment at least one active ingredient selected from group B-5 is used on a plant comprising EVENT1 or plant parts thereof, preferably for increasing yield.

In one embodiment at least one active ingredient selected from group I-1 is used on a plant comprising EVENT1 or plant parts thereof, preferably for increasing yield.

In one embodiment at least one active ingredient selected from group I-2 is used on a plant comprising EVENT1 or plant parts thereof, preferably for increasing yield.

In one embodiment at least one active ingredient selected from group I-3 is used on a plant comprising EVENT1 or plant parts thereof, preferably for increasing yield.

In one embodiment at least one active ingredient selected from group I-4 is used on a plant comprising EVENT1 or plant parts thereof, preferably for increasing yield.

In one embodiment at least one active ingredient selected from group I-5 is used on a plant comprising EVENT1 or plant parts thereof, preferably for increasing yield.

In one embodiment at least one active ingredient selected from group I-6 is used on a plant comprising EVENT1 or plant parts thereof, preferably for increasing yield.

In one embodiment at least one active ingredient selected from group F-1 is used on a plant comprising EVENT1 or plant parts thereof, preferably for increasing yield.

In one embodiment at least one active ingredient selected from group F-2 is used on a plant comprising EVENT1 or plant parts thereof, preferably for increasing yield.

In one embodiment at least one active ingredient selected from group F-3 is used on a plant comprising EVENT1 or plant parts thereof, preferably for increasing yield.

In one embodiment at least one active ingredient selected from group F-4 is used on a plant comprising EVENT1 or plant parts thereof, preferably for increasing yield.

In one embodiment at least one active ingredient selected from group F-5 is used on a plant comprising EVENT1 or plant parts thereof, preferably for increasing yield.

In one embodiment at least one active ingredient selected from group F-6 is used on a plant comprising EVENT1 or plant parts thereof, preferably for increasing yield.

In one embodiment at least one active ingredient selected from group SG-1 is used on a plant comprising EVENT1 or plant parts thereof, preferably for increasing yield.

In one embodiment at least one active ingredient selected from group SG-2 is used on a plant comprising EVENT1 or plant parts thereof, preferably for increasing yield.

In one embodiment at least one active ingredient selected from group SG-3 is used on a plant comprising EVENT1 or plant parts thereof, preferably for increasing yield.

In one embodiment at least one active ingredient selected from group SG-4 is used on a plant comprising EVENT1 or plant parts thereof, preferably for increasing yield.

In one embodiment at least one active ingredient selected from group SG-5 is used on a plant comprising EVENT1 or plant parts thereof, preferably for increasing yield.

In one embodiment at least one active ingredient selected from group SG-6 is used on a plant comprising EVENT1 or plant parts thereof, preferably for increasing yield.

In one embodiment at least one active ingredient selected from group SG-7 is used on a plant comprising EVENT1 or plant parts thereof, preferably for increasing yield.

In one embodiment at least one active ingredient selected from group SG-8 is used on a plant comprising EVENT1 or plant parts thereof, preferably for increasing yield.

In one embodiment at least one active ingredient selected from group H-1 is used on a plant comprising EVENT2 or plant parts thereof, preferably for increasing yield.

In one embodiment at least one active ingredient selected from group H-2 is used on a plant comprising EVENT2 or plant parts thereof, preferably for increasing yield.

In one embodiment at least one active ingredient selected from group H-3 is used on a plant comprising EVENT2 or plant parts thereof, preferably for increasing yield.

In one embodiment at least one active ingredient selected from group H-4 is used on a plant comprising EVENT2 or plant parts thereof, preferably for increasing yield.

In one embodiment at least one active ingredient selected from group H-5 is used on a plant comprising EVENT2 or plant parts thereof, preferably for increasing yield.

In one embodiment at least one active ingredient selected from group P-1 is used on a plant comprising EVENT2 or plant parts thereof, preferably for increasing yield.

In one embodiment at least one active ingredient selected from group SAF-1 is used on a plant comprising EVENT2 or plant parts thereof, preferably for increasing yield.

In one embodiment at least one active ingredient selected from group SAF-2 is used on a plant comprising EVENT2 or plant parts thereof, preferably for increasing yield.

In one embodiment at least one active ingredient selected from group SAF-3 is used on a plant comprising EVENT2 or plant parts thereof, preferably for increasing yield.

In one embodiment at least one active ingredient selected from group SAF-4 is used on a plant comprising EVENT2 or plant parts thereof, preferably for increasing yield.

In one embodiment at least one active ingredient selected from group B-1 is used on a plant comprising EVENT2 or plant parts thereof, preferably for increasing yield.

In one embodiment at least one active ingredient selected from group B-2 is used on a plant comprising EVENT2 or plant parts thereof, preferably for increasing yield.

In one embodiment at least one active ingredient selected from group B-3 is used on a plant comprising EVENT2 or plant parts thereof, preferably for increasing yield.

In one embodiment at least one active ingredient selected from group B-4 is used on a plant comprising EVENT2 or plant parts thereof, preferably for increasing yield.

In one embodiment at least one active ingredient selected from group B-5 is used on a plant comprising EVENT2 or plant parts thereof, preferably for increasing yield.

In one embodiment at least one active ingredient selected from group I-1 is used on a plant comprising EVENT2 or plant parts thereof, preferably for increasing yield.

In one embodiment at least one active ingredient selected from group I-2 is used on a plant comprising EVENT2 or plant parts thereof, preferably for increasing yield.

In one embodiment at least one active ingredient selected from group I-3 is used on a plant comprising EVENT2 or plant parts thereof, preferably for increasing yield.

In one embodiment at least one active ingredient selected from group I-4 is used on a plant comprising EVENT2 or plant parts thereof, preferably for increasing yield.

In one embodiment at least one active ingredient selected from group I-5 is used on a plant comprising EVENT2 or plant parts thereof, preferably for increasing yield.

In one embodiment at least one active ingredient selected from group I-6 is used on a plant comprising EVENT2 or plant parts thereof, preferably for increasing yield.

In one embodiment at least one active ingredient selected from group F-1 is used on a plant comprising EVENT2 or plant parts thereof, preferably for increasing yield.

In one embodiment at least one active ingredient selected from group F-2 is used on a plant comprising EVENT2 or plant parts thereof, preferably for increasing yield.

In one embodiment at least one active ingredient selected from group F-3 is used on a plant comprising EVENT2 or plant parts thereof, preferably for increasing yield.

In one embodiment at least one active ingredient selected from group F-4 is used on a plant comprising EVENT2 or plant parts thereof, preferably for increasing yield.

In one embodiment at least one active ingredient selected from group F-5 is used on a plant comprising EVENT2 or plant parts thereof, preferably for increasing yield.

In one embodiment at least one active ingredient selected from group F-6 is used on a plant comprising EVENT2 or plant parts thereof, preferably for increasing yield.

In one embodiment at least one active ingredient selected from group SG-1 is used on a plant comprising EVENT2 or plant parts thereof, preferably for increasing yield.

In one embodiment at least one active ingredient selected from group SG-2 is used on a plant comprising EVENT2 or plant parts thereof, preferably for increasing yield.

In one embodiment at least one active ingredient selected from group SG-3 is used on a plant comprising EVENT2 or plant parts thereof, preferably for increasing yield.

In one embodiment at least one active ingredient selected from group SG-4 is used on a plant comprising EVENT2 or plant parts thereof, preferably for increasing yield.

In one embodiment at least one active ingredient selected from group SG-5 is used on a plant comprising EVENT2 or plant parts thereof, preferably for increasing yield.

In one embodiment at least one active ingredient selected from group SG-6 is used on a plant comprising EVENT2 or plant parts thereof, preferably for increasing yield.

In one embodiment at least one active ingredient selected from group SG-7 is used on a plant comprising EVENT2 or plant parts thereof, preferably for increasing yield.

In one embodiment at least one active ingredient selected from group SG-8 is used on a plant comprising EVENT2 or plant parts thereof, preferably for increasing yield.

In one embodiment at least one active ingredient selected from group H-1 is used on a plant comprising EVENT3 or plant parts thereof, preferably for increasing yield.

In one embodiment at least one active ingredient selected from group H-2 is used on a plant comprising EVENT3 or plant parts thereof, preferably for increasing yield.

In one embodiment at least one active ingredient selected from group H-3 is used on a plant comprising EVENT3 or plant parts thereof, preferably for increasing yield.

In one embodiment at least one active ingredient selected from group H-4 is used on a plant comprising EVENT3 or plant parts thereof, preferably for increasing yield.

In one embodiment at least one active ingredient selected from group H-5 is used on a plant comprising EVENT3 or plant parts thereof, preferably for increasing yield.

In one embodiment at least one active ingredient selected from group P-1 is used on a plant comprising EVENT3 or plant parts thereof, preferably for increasing yield.

In one embodiment at least one active ingredient selected from group SAF-1 is used on a plant comprising EVENT3 or plant parts thereof, preferably for increasing yield.

In one embodiment at least one active ingredient selected from group SAF-2 is used on a plant comprising EVENT3 or plant parts thereof, preferably for increasing yield.

In one embodiment at least one active ingredient selected from group SAF-3 is used on a plant comprising EVENT3 or plant parts thereof, preferably for increasing yield.

In one embodiment at least one active ingredient selected from group SAF-4 is used on a plant comprising EVENT3 or plant parts thereof, preferably for increasing yield.

In one embodiment at least one active ingredient selected from group B-1 is used on a plant comprising EVENT3 or plant parts thereof, preferably for increasing yield.

In one embodiment at least one active ingredient selected from group B-2 is used on a plant comprising EVENT3 or plant parts thereof, preferably for increasing yield.

In one embodiment at least one active ingredient selected from group B-3 is used on a plant comprising EVENT3 or plant parts thereof, preferably for increasing yield.

In one embodiment at least one active ingredient selected from group B-4 is used on a plant comprising EVENT3 or plant parts thereof, preferably for increasing yield.

In one embodiment at least one active ingredient selected from group B-5 is used on a plant comprising EVENT3 or plant parts thereof, preferably for increasing yield.

In one embodiment at least one active ingredient selected from group I-1 is used on a plant comprising EVENT3 or plant parts thereof, preferably for increasing yield.

In one embodiment at least one active ingredient selected from group I-2 is used on a plant comprising EVENT3 or plant parts thereof, preferably for increasing yield.

In one embodiment at least one active ingredient selected from group I-3 is used on a plant comprising EVENT3 or plant parts thereof, preferably for increasing yield.

In one embodiment at least one active ingredient selected from group I-4 is used on a plant comprising EVENT3 or plant parts thereof, preferably for increasing yield.

In one embodiment at least one active ingredient selected from group I-5 is used on a plant comprising EVENT3 or plant parts thereof, preferably for increasing yield.

In one embodiment at least one active ingredient selected from group I-6 is used on a plant comprising EVENT3 or plant parts thereof, preferably for increasing yield.

In one embodiment at least one active ingredient selected from group F-1 is used on a plant comprising EVENT3 or plant parts thereof, preferably for increasing yield.

In one embodiment at least one active ingredient selected from group F-2 is used on a plant comprising EVENT3 or plant parts thereof, preferably for increasing yield.

In one embodiment at least one active ingredient selected from group F-3 is used on a plant comprising EVENT3 or plant parts thereof, preferably for increasing yield.

In one embodiment at least one active ingredient selected from group F-4 is used on a plant comprising EVENT3 or plant parts thereof, preferably for increasing yield.

In one embodiment at least one active ingredient selected from group F-5 is used on a plant comprising EVENT3 or plant parts thereof, preferably for increasing yield.

In one embodiment at least one active ingredient selected from group F-6 is used on a plant comprising EVENT3 or plant parts thereof, preferably for increasing yield.

In one embodiment at least one active ingredient selected from group SG-1 is used on a plant comprising EVENT3 or plant parts thereof, preferably for increasing yield.

In one embodiment at least one active ingredient selected from group SG-2 is used on a plant comprising EVENT3 or plant parts thereof, preferably for increasing yield.

In one embodiment at least one active ingredient selected from group SG-3 is used on a plant comprising EVENT3 or plant parts thereof, preferably for increasing yield.

In one embodiment at least one active ingredient selected from group SG-4 is used on a plant comprising EVENT3 or plant parts thereof, preferably for increasing yield.

In one embodiment at least one active ingredient selected from group SG-5 is used on a plant comprising EVENT3 or plant parts thereof, preferably for increasing yield.

In one embodiment at least one active ingredient selected from group SG-6 is used on a plant comprising EVENT3 or plant parts thereof, preferably for increasing yield.

In one embodiment at least one active ingredient selected from group SG-7 is used on a plant comprising EVENT3 or plant parts thereof, preferably for increasing yield.

In one embodiment at least one active ingredient selected from group SG-8 is used on a plant comprising EVENT3 or plant parts thereof, preferably for increasing yield.

In one embodiment at least one active ingredient selected from group H-1 is used on a plant comprising EVENT4 or plant parts thereof, preferably for increasing yield.

In one embodiment at least one active ingredient selected from group H-2 is used on a plant comprising EVENT4 or plant parts thereof, preferably for increasing yield.

In one embodiment at least one active ingredient selected from group H-3 is used on a plant comprising EVENT4 or plant parts thereof, preferably for increasing yield.

In one embodiment at least one active ingredient selected from group H-4 is used on a plant comprising EVENT4 or plant parts thereof, preferably for increasing yield.

In one embodiment at least one active ingredient selected from group H-5 is used on a plant comprising EVENT4 or plant parts thereof, preferably for increasing yield.

In one embodiment at least one active ingredient selected from group P-1 is used on a plant comprising EVENT4 or plant parts thereof, preferably for increasing yield.

In one embodiment at least one active ingredient selected from group SAF-1 is used on a plant comprising EVENT4 or plant parts thereof, preferably for increasing yield.

In one embodiment at least one active ingredient selected from group SAF-2 is used on a plant comprising EVENT4 or plant parts thereof, preferably for increasing yield.

In one embodiment at least one active ingredient selected from group SAF-3 is used on a plant comprising EVENT4 or plant parts thereof, preferably for increasing yield.

In one embodiment at least one active ingredient selected from group SAF-4 is used on a plant comprising EVENT4 or plant parts thereof, preferably for increasing yield.

In one embodiment at least one active ingredient selected from group B-1 is used on a plant comprising EVENT4 or plant parts thereof, preferably for increasing yield.

In one embodiment at least one active ingredient selected from group B-2 is used on a plant comprising EVENT4 or plant parts thereof, preferably for increasing yield.

In one embodiment at least one active ingredient selected from group B-3 is used on a plant comprising EVENT4 or plant parts thereof, preferably for increasing yield.

In one embodiment at least one active ingredient selected from group B-4 is used on a plant comprising EVENT4 or plant parts thereof, preferably for increasing yield.

In one embodiment at least one active ingredient selected from group B-5 is used on a plant comprising EVENT4 or plant parts thereof, preferably for increasing yield.

In one embodiment at least one active ingredient selected from group I-1 is used on a plant comprising EVENT4 or plant parts thereof, preferably for increasing yield.

In one embodiment at least one active ingredient selected from group I-2 is used on a plant comprising EVENT4 or plant parts thereof, preferably for increasing yield.

In one embodiment at least one active ingredient selected from group I-3 is used on a plant comprising EVENT4 or plant parts thereof, preferably for increasing yield.

In one embodiment at least one active ingredient selected from group I-4 is used on a plant comprising EVENT4 or plant parts thereof, preferably for increasing yield.

In one embodiment at least one active ingredient selected from group I-5 is used on a plant comprising EVENT4 or plant parts thereof, preferably for increasing yield.

In one embodiment at least one active ingredient selected from group I-6 is used on a plant comprising EVENT4 or plant parts thereof, preferably for increasing yield.

In one embodiment at least one active ingredient selected from group F-1 is used on a plant comprising EVENT4 or plant parts thereof, preferably for increasing yield.

In one embodiment at least one active ingredient selected from group F-2 is used on a plant comprising EVENT4 or plant parts thereof, preferably for increasing yield.

In one embodiment at least one active ingredient selected from group F-3 is used on a plant comprising EVENT4 or plant parts thereof, preferably for increasing yield.

In one embodiment at least one active ingredient selected from group F-4 is used on a plant comprising EVENT4 or plant parts thereof, preferably for increasing yield.

In one embodiment at least one active ingredient selected from group F-5 is used on a plant comprising EVENT4 or plant parts thereof, preferably for increasing yield.

In one embodiment at least one active ingredient selected from group F-6 is used on a plant comprising EVENT4 or plant parts thereof, preferably for increasing yield.

In one embodiment at least one active ingredient selected from group SG-1 is used on a plant comprising EVENT4 or plant parts thereof, preferably for increasing yield.

In one embodiment at least one active ingredient selected from group SG-2 is used on a plant comprising EVENT4 or plant parts thereof, preferably for increasing yield.

In one embodiment at least one active ingredient selected from group SG-3 is used on a plant comprising EVENT4 or plant parts thereof, preferably for increasing yield.

In one embodiment at least one active ingredient selected from group SG-4 is used on a plant comprising EVENT4 or plant parts thereof, preferably for increasing yield.

In one embodiment at least one active ingredient selected from group SG-5 is used on a plant comprising EVENT4 or plant parts thereof, preferably for increasing yield.

In one embodiment at least one active ingredient selected from group SG-6 is used on a plant comprising EVENT4 or plant parts thereof, preferably for increasing yield.

In one embodiment at least one active ingredient selected from group SG-7 is used on a plant comprising EVENT4 or plant parts thereof, preferably for increasing yield.

In one embodiment at least one active ingredient selected from group SG-8 is used on a plant comprising EVENT4 or plant parts thereof, preferably for increasing yield.

In one embodiment at least one active ingredient selected from group H-1 is used on a plant comprising EVENT5 or plant parts thereof, preferably for increasing yield.

In one embodiment at least one active ingredient selected from group H-2 is used on a plant comprising EVENT5 or plant parts thereof, preferably for increasing yield.

In one embodiment at least one active ingredient selected from group H-3 is used on a plant comprising EVENT5 or plant parts thereof, preferably for increasing yield.

In one embodiment at least one active ingredient selected from group H-4 is used on a plant comprising EVENT5 or plant parts thereof, preferably for increasing yield.

In one embodiment at least one active ingredient selected from group H-5 is used on a plant comprising EVENT5 or plant parts thereof, preferably for increasing yield.

In one embodiment at least one active ingredient selected from group P-1 is used on a plant comprising EVENT5 or plant parts thereof, preferably for increasing yield.

In one embodiment at least one active ingredient selected from group SAF-1 is used on a plant comprising EVENT5 or plant parts thereof, preferably for increasing yield.

In one embodiment at least one active ingredient selected from group SAF-2 is used on a plant comprising EVENT5 or plant parts thereof, preferably for increasing yield.

In one embodiment at least one active ingredient selected from group SAF-3 is used on a plant comprising EVENT5 or plant parts thereof, preferably for increasing yield.

In one embodiment at least one active ingredient selected from group SAF-4 is used on a plant comprising EVENT5 or plant parts thereof, preferably for increasing yield.

In one embodiment at least one active ingredient selected from group B-1 is used on a plant comprising EVENT5 or plant parts thereof, preferably for increasing yield.

In one embodiment at least one active ingredient selected from group B-2 is used on a plant comprising EVENT5 or plant parts thereof, preferably for increasing yield.

In one embodiment at least one active ingredient selected from group B-3 is used on a plant comprising EVENT5 or plant parts thereof, preferably for increasing yield.

In one embodiment at least one active ingredient selected from group B-4 is used on a plant comprising EVENT5 or plant parts thereof, preferably for increasing yield.

In one embodiment at least one active ingredient selected from group B-5 is used on a plant comprising EVENT5 or plant parts thereof, preferably for increasing yield.

In one embodiment at least one active ingredient selected from group I-1 is used on a plant comprising EVENT5 or plant parts thereof, preferably for increasing yield.

In one embodiment at least one active ingredient selected from group I-2 is used on a plant comprising EVENT5 or plant parts thereof, preferably for increasing yield.

In one embodiment at least one active ingredient selected from group I-3 is used on a plant comprising EVENT5 or plant parts thereof, preferably for increasing yield.

In one embodiment at least one active ingredient selected from group I-4 is used on a plant comprising EVENT5 or plant parts thereof, preferably for increasing yield.

In one embodiment at least one active ingredient selected from group I-5 is used on a plant comprising EVENT5 or plant parts thereof, preferably for increasing yield.

In one embodiment at least one active ingredient selected from group I-6 is used on a plant comprising EVENT5 or plant parts thereof, preferably for increasing yield.

In one embodiment at least one active ingredient selected from group F-1 is used on a plant comprising EVENT5 or plant parts thereof, preferably for increasing yield.

In one embodiment at least one active ingredient selected from group F-2 is used on a plant comprising EVENT5 or plant parts thereof, preferably for increasing yield.

In one embodiment at least one active ingredient selected from group F-3 is used on a plant comprising EVENT5 or plant parts thereof, preferably for increasing yield.

In one embodiment at least one active ingredient selected from group F-4 is used on a plant comprising EVENT5 or plant parts thereof, preferably for increasing yield.

In one embodiment at least one active ingredient selected from group F-5 is used on a plant comprising EVENT5 or plant parts thereof, preferably for increasing yield.

In one embodiment at least one active ingredient selected from group F-6 is used on a plant comprising EVENT5 or plant parts thereof, preferably for increasing yield.

In one embodiment at least one active ingredient selected from group SG-1 is used on a plant comprising EVENT5 or plant parts thereof, preferably for increasing yield.

In one embodiment at least one active ingredient selected from group SG-2 is used on a plant comprising EVENT5 or plant parts thereof, preferably for increasing yield.

In one embodiment at least one active ingredient selected from group SG-3 is used on a plant comprising EVENT5 or plant parts thereof, preferably for increasing yield.

In one embodiment at least one active ingredient selected from group SG-4 is used on a plant comprising EVENT5 or plant parts thereof, preferably for increasing yield.

In one embodiment at least one active ingredient selected from group SG-5 is used on a plant comprising EVENT5 or plant parts thereof, preferably for increasing yield.

In one embodiment at least one active ingredient selected from group SG-6 is used on a plant comprising EVENT5 or plant parts thereof, preferably for increasing yield.

In one embodiment at least one active ingredient selected from group SG-7 is used on a plant comprising EVENT5 or plant parts thereof, preferably for increasing yield.

In one embodiment at least one active ingredient selected from group SG-8 is used on a plant comprising EVENT5 or plant parts thereof, preferably for increasing yield.

In one embodiment at least one active ingredient selected from group H-1 is used on a plant comprising EVENT6 or plant parts thereof, preferably for increasing yield.

In one embodiment at least one active ingredient selected from group H-2 is used on a plant comprising EVENT6 or plant parts thereof, preferably for increasing yield.

In one embodiment at least one active ingredient selected from group H-3 is used on a plant comprising EVENT6 or plant parts thereof, preferably for increasing yield.

In one embodiment at least one active ingredient selected from group H-4 is used on a plant comprising EVENT6 or plant parts thereof, preferably for increasing yield.

In one embodiment at least one active ingredient selected from group H-5 is used on a plant comprising EVENT6 or plant parts thereof, preferably for increasing yield.

In one embodiment at least one active ingredient selected from group P-1 is used on a plant comprising EVENT6 or plant parts thereof, preferably for increasing yield.

In one embodiment at least one active ingredient selected from group SAF-1 is used on a plant comprising EVENT6 or plant parts thereof, preferably for increasing yield.

In one embodiment at least one active ingredient selected from group SAF-2 is used on a plant comprising EVENT6 or plant parts thereof, preferably for increasing yield.

In one embodiment at least one active ingredient selected from group SAF-3 is used on a plant comprising EVENT6 or plant parts thereof, preferably for increasing yield.

In one embodiment at least one active ingredient selected from group SAF-4 is used on a plant comprising EVENT6 or plant parts thereof, preferably for increasing yield.

In one embodiment at least one active ingredient selected from group B-1 is used on a plant comprising EVENT6 or plant parts thereof, preferably for increasing yield.

In one embodiment at least one active ingredient selected from group B-2 is used on a plant comprising EVENT6 or plant parts thereof, preferably for increasing yield.

In one embodiment at least one active ingredient selected from group B-3 is used on a plant comprising EVENT6 or plant parts thereof, preferably for increasing yield.

In one embodiment at least one active ingredient selected from group B-4 is used on a plant comprising EVENT6 or plant parts thereof, preferably for increasing yield.

In one embodiment at least one active ingredient selected from group B-5 is used on a plant comprising EVENT6 or plant parts thereof, preferably for increasing yield.

In one embodiment at least one active ingredient selected from group I-1 is used on a plant comprising EVENT6 or plant parts thereof, preferably for increasing yield.

In one embodiment at least one active ingredient selected from group I-2 is used on a plant comprising EVENT6 or plant parts thereof, preferably for increasing yield.

In one embodiment at least one active ingredient selected from group I-3 is used on a plant comprising EVENT6 or plant parts thereof, preferably for increasing yield.

In one embodiment at least one active ingredient selected from group I-4 is used on a plant comprising EVENT6 or plant parts thereof, preferably for increasing yield.

In one embodiment at least one active ingredient selected from group I-5 is used on a plant comprising EVENT6 or plant parts thereof, preferably for increasing yield.

In one embodiment at least one active ingredient selected from group I-6 is used on a plant comprising EVENT6 or plant parts thereof, preferably for increasing yield.

In one embodiment at least one active ingredient selected from group F-1 is used on a plant comprising EVENT6 or plant parts thereof, preferably for increasing yield.

In one embodiment at least one active ingredient selected from group F-2 is used on a plant comprising EVENT6 or plant parts thereof, preferably for increasing yield.

In one embodiment at least one active ingredient selected from group F-3 is used on a plant comprising EVENT6 or plant parts thereof, preferably for increasing yield.

In one embodiment at least one active ingredient selected from group F-4 is used on a plant comprising EVENT6 or plant parts thereof, preferably for increasing yield.

In one embodiment at least one active ingredient selected from group F-5 is used on a plant comprising EVENT6 or plant parts thereof, preferably for increasing yield.

In one embodiment at least one active ingredient selected from group F-6 is used on a plant comprising EVENT6 or plant parts thereof, preferably for increasing yield.

In one embodiment at least one active ingredient selected from group SG-1 is used on a plant comprising EVENT6 or plant parts thereof, preferably for increasing yield.

In one embodiment at least one active ingredient selected from group SG-2 is used on a plant comprising EVENT6 or plant parts thereof, preferably for increasing yield.

In one embodiment at least one active ingredient selected from group SG-3 is used on a plant comprising EVENT6 or plant parts thereof, preferably for increasing yield.

In one embodiment at least one active ingredient selected from group SG-4 is used on a plant comprising EVENT6 or plant parts thereof, preferably for increasing yield.

In one embodiment at least one active ingredient selected from group SG-5 is used on a plant comprising EVENT6 or plant parts thereof, preferably for increasing yield.

In one embodiment at least one active ingredient selected from group SG-6 is used on a plant comprising EVENT6 or plant parts thereof, preferably for increasing yield.

In one embodiment at least one active ingredient selected from group SG-7 is used on a plant comprising EVENT6 or plant parts thereof, preferably for increasing yield.

In one embodiment at least one active ingredient selected from group SG-8 is used on a plant comprising EVENT6 or plant parts thereof, preferably for increasing yield.

In one embodiment at least one active ingredient selected from group H-1 is used on a plant comprising EVENT7 or plant parts thereof, preferably for increasing yield.

In one embodiment at least one active ingredient selected from group H-2 is used on a plant comprising EVENT7 or plant parts thereof, preferably for increasing yield.

In one embodiment at least one active ingredient selected from group H-3 is used on a plant comprising EVENT7 or plant parts thereof, preferably for increasing yield.

In one embodiment at least one active ingredient selected from group H-4 is used on a plant comprising EVENT7 or plant parts thereof, preferably for increasing yield.

In one embodiment at least one active ingredient selected from group H-5 is used on a plant comprising EVENT7 or plant parts thereof, preferably for increasing yield.

In one embodiment at least one active ingredient selected from group P-1 is used on a plant comprising EVENT7 or plant parts thereof, preferably for increasing yield.

In one embodiment at least one active ingredient selected from group SAF-1 is used on a plant comprising EVENT7 or plant parts thereof, preferably for increasing yield.

In one embodiment at least one active ingredient selected from group SAF-2 is used on a plant comprising EVENT7 or plant parts thereof, preferably for increasing yield.

In one embodiment at least one active ingredient selected from group SAF-3 is used on a plant comprising EVENT7 or plant parts thereof, preferably for increasing yield.

In one embodiment at least one active ingredient selected from group SAF-4 is used on a plant comprising EVENT7 or plant parts thereof, preferably for increasing yield.

In one embodiment at least one active ingredient selected from group B-1 is used on a plant comprising EVENT7 or plant parts thereof, preferably for increasing yield.

In one embodiment at least one active ingredient selected from group B-2 is used on a plant comprising EVENT7 or plant parts thereof, preferably for increasing yield.

In one embodiment at least one active ingredient selected from group B-3 is used on a plant comprising EVENT7 or plant parts thereof, preferably for increasing yield.

In one embodiment at least one active ingredient selected from group B-4 is used on a plant comprising EVENT7 or plant parts thereof, preferably for increasing yield.

In one embodiment at least one active ingredient selected from group B-5 is used on a plant comprising EVENT7 or plant parts thereof, preferably for increasing yield.

In one embodiment at least one active ingredient selected from group I-1 is used on a plant comprising EVENT7 or plant parts thereof, preferably for increasing yield.

In one embodiment at least one active ingredient selected from group I-2 is used on a plant comprising EVENT7 or plant parts thereof, preferably for increasing yield.

In one embodiment at least one active ingredient selected from group I-3 is used on a plant comprising EVENT7 or plant parts thereof, preferably for increasing yield.

In one embodiment at least one active ingredient selected from group I-4 is used on a plant comprising EVENT7 or plant parts thereof, preferably for increasing yield.

In one embodiment at least one active ingredient selected from group I-5 is used on a plant comprising EVENT7 or plant parts thereof, preferably for increasing yield.

In one embodiment at least one active ingredient selected from group I-6 is used on a plant comprising EVENT7 or plant parts thereof, preferably for increasing yield.

In one embodiment at least one active ingredient selected from group F-1 is used on a plant comprising EVENT7 or plant parts thereof, preferably for increasing yield.

In one embodiment at least one active ingredient selected from group F-2 is used on a plant comprising EVENT7 or plant parts thereof, preferably for increasing yield.

In one embodiment at least one active ingredient selected from group F-3 is used on a plant comprising EVENT7 or plant parts thereof, preferably for increasing yield.

In one embodiment at least one active ingredient selected from group F-4 is used on a plant comprising EVENT7 or plant parts thereof, preferably for increasing yield.

In one embodiment at least one active ingredient selected from group F-5 is used on a plant comprising EVENT7 or plant parts thereof, preferably for increasing yield.

In one embodiment at least one active ingredient selected from group F-6 is used on a plant comprising EVENT7 or plant parts thereof, preferably for increasing yield.

In one embodiment at least one active ingredient selected from group SG-1 is used on a plant comprising EVENT7 or plant parts thereof, preferably for increasing yield.

In one embodiment at least one active ingredient selected from group SG-2 is used on a plant comprising EVENT7 or plant parts thereof, preferably for increasing yield.

In one embodiment at least one active ingredient selected from group SG-3 is used on a plant comprising EVENT7 or plant parts thereof, preferably for increasing yield.

In one embodiment at least one active ingredient selected from group SG-4 is used on a plant comprising EVENT7 or plant parts thereof, preferably for increasing yield.

In one embodiment at least one active ingredient selected from group SG-5 is used on a plant comprising EVENT7 or plant parts thereof, preferably for increasing yield.

In one embodiment at least one active ingredient selected from group SG-6 is used on a plant comprising EVENT7 or plant parts thereof, preferably for increasing yield.

In one embodiment at least one active ingredient selected from group SG-7 is used on a plant comprising EVENT7 or plant parts thereof, preferably for increasing yield.

In one embodiment at least one active ingredient selected from group SG-8 is used on a plant comprising EVENT7 or plant parts thereof, preferably for increasing yield.

In one embodiment at least one active ingredient selected from group H-1 is used on a plant comprising ALLELE1 or plant parts thereof, preferably for increasing yield.

In one embodiment at least one active ingredient selected from group H-2 is used on a plant comprising ALLELE1 or plant parts thereof, preferably for increasing yield.

In one embodiment at least one active ingredient selected from group H-3 is used on a plant comprising ALLELE1 or plant parts thereof, preferably for increasing yield.

In one embodiment at least one active ingredient selected from group H-4 is used on a plant comprising ALLELE1 or plant parts thereof, preferably for increasing yield.

In one embodiment at least one active ingredient selected from group H-5 is used on a plant comprising ALLELE1 or plant parts thereof, preferably for increasing yield.

In one embodiment at least one active ingredient selected from group P-1 is used on a plant comprising ALLELE1 or plant parts thereof, preferably for increasing yield.

In one embodiment at least one active ingredient selected from group SAF-1 is used on a plant comprising ALLELE1 or plant parts thereof, preferably for increasing yield.

In one embodiment at least one active ingredient selected from group SAF-2 is used on a plant comprising ALLELE1 or plant parts thereof, preferably for increasing yield.

In one embodiment at least one active ingredient selected from group SAF-3 is used on a plant comprising ALLELE1 or plant parts thereof, preferably for increasing yield.

In one embodiment at least one active ingredient selected from group SAF-4 is used on a plant comprising ALLELE1 or plant parts thereof, preferably for increasing yield.

In one embodiment at least one active ingredient selected from group B-1 is used on a plant comprising ALLELE1 or plant parts thereof, preferably for increasing yield.

In one embodiment at least one active ingredient selected from group B-2 is used on a plant comprising ALLELE1 or plant parts thereof, preferably for increasing yield.

In one embodiment at least one active ingredient selected from group B-3 is used on a plant comprising ALLELE1 or plant parts thereof, preferably for increasing yield.

In one embodiment at least one active ingredient selected from group B-4 is used on a plant comprising ALLELE1 or plant parts thereof, preferably for increasing yield.

In one embodiment at least one active ingredient selected from group B-5 is used on a plant comprising ALLELE1 or plant parts thereof, preferably for increasing yield.

In one embodiment at least one active ingredient selected from group I-1 is used on a plant comprising ALLELE1 or plant parts thereof, preferably for increasing yield.

In one embodiment at least one active ingredient selected from group I-2 is used on a plant comprising ALLELE1 or plant parts thereof, preferably for increasing yield.

In one embodiment at least one active ingredient selected from group I-3 is used on a plant comprising ALLELE1 or plant parts thereof, preferably for increasing yield.

In one embodiment at least one active ingredient selected from group I-4 is used on a plant comprising ALLELE1 or plant parts thereof, preferably for increasing yield.

In one embodiment at least one active ingredient selected from group I-5 is used on a plant comprising ALLELE1 or plant parts thereof, preferably for increasing yield.

In one embodiment at least one active ingredient selected from group I-6 is used on a plant comprising ALLELE1 or plant parts thereof, preferably for increasing yield.

In one embodiment at least one active ingredient selected from group F-1 is used on a plant comprising ALLELE1 or plant parts thereof, preferably for increasing yield.

In one embodiment at least one active ingredient selected from group F-2 is used on a plant comprising ALLELE1 or plant parts thereof, preferably for increasing yield.

In one embodiment at least one active ingredient selected from group F-3 is used on a plant comprising ALLELE1 or plant parts thereof, preferably for increasing yield.

In one embodiment at least one active ingredient selected from group F-4 is used on a plant comprising ALLELE1 or plant parts thereof, preferably for increasing yield.

In one embodiment at least one active ingredient selected from group F-5 is used on a plant comprising ALLELE1 or plant parts thereof, preferably for increasing yield.

In one embodiment at least one active ingredient selected from group F-6 is used on a plant comprising ALLELE1 or plant parts thereof, preferably for increasing yield.

In one embodiment at least one active ingredient selected from group SG-1 is used on a plant comprising ALLELE1 or plant parts thereof, preferably for increasing yield.

In one embodiment at least one active ingredient selected from group SG-2 is used on a plant comprising ALLELE1 or plant parts thereof, preferably for increasing yield.

In one embodiment at least one active ingredient selected from group SG-3 is used on a plant comprising ALLELE1 or plant parts thereof, preferably for increasing yield.

In one embodiment at least one active ingredient selected from group SG-4 is used on a plant comprising ALLELE1 or plant parts thereof, preferably for increasing yield.

In one embodiment at least one active ingredient selected from group SG-5 is used on a plant comprising ALLELE1 or plant parts thereof, preferably for increasing yield.

In one embodiment at least one active ingredient selected from group SG-6 is used on a plant comprising ALLELE1 or plant parts thereof, preferably for increasing yield.

In one embodiment at least one active ingredient selected from group SG-7 is used on a plant comprising ALLELE1 or plant parts thereof, preferably for increasing yield.

In one embodiment at least one active ingredient selected from group SG-8 is used on a plant comprising ALLELE1 or plant parts thereof, preferably for increasing yield.

In one embodiment at least one active ingredient selected from group H-1 is used on a plant comprising ALLELE2 or plant parts thereof, preferably for increasing yield.

In one embodiment at least one active ingredient selected from group H-2 is used on a plant comprising ALLELE2 or plant parts thereof, preferably for increasing yield.

In one embodiment at least one active ingredient selected from group H-3 is used on a plant comprising ALLELE2 or plant parts thereof, preferably for increasing yield.

In one embodiment at least one active ingredient selected from group H-4 is used on a plant comprising ALLELE2 or plant parts thereof, preferably for increasing yield.

In one embodiment at least one active ingredient selected from group H-5 is used on a plant comprising ALLELE2 or plant parts thereof, preferably for increasing yield.

In one embodiment at least one active ingredient selected from group P-1 is used on a plant comprising ALLELE2 or plant parts thereof, preferably for increasing yield.

In one embodiment at least one active ingredient selected from group SAF-1 is used on a plant comprising ALLELE2 or plant parts thereof, preferably for increasing yield.

In one embodiment at least one active ingredient selected from group SAF-2 is used on a plant comprising ALLELE2 or plant parts thereof, preferably for increasing yield.

In one embodiment at least one active ingredient selected from group SAF-3 is used on a plant comprising ALLELE2 or plant parts thereof, preferably for increasing yield.

In one embodiment at least one active ingredient selected from group SAF-4 is used on a plant comprising ALLELE2 or plant parts thereof, preferably for increasing yield.

In one embodiment at least one active ingredient selected from group B-1 is used on a plant comprising ALLELE2 or plant parts thereof, preferably for increasing yield.

In one embodiment at least one active ingredient selected from group B-2 is used on a plant comprising ALLELE2 or plant parts thereof, preferably for increasing yield.

In one embodiment at least one active ingredient selected from group B-3 is used on a plant comprising ALLELE2 or plant parts thereof, preferably for increasing yield.

In one embodiment at least one active ingredient selected from group B-4 is used on a plant comprising ALLELE2 or plant parts thereof, preferably for increasing yield.

In one embodiment at least one active ingredient selected from group B-5 is used on a plant comprising ALLELE2 or plant parts thereof, preferably for increasing yield.

In one embodiment at least one active ingredient selected from group I-1 is used on a plant comprising ALLELE2 or plant parts thereof, preferably for increasing yield.

In one embodiment at least one active ingredient selected from group I-2 is used on a plant comprising ALLELE2 or plant parts thereof, preferably for increasing yield.

In one embodiment at least one active ingredient selected from group I-3 is used on a plant comprising ALLELE2 or plant parts thereof, preferably for increasing yield.

In one embodiment at least one active ingredient selected from group I-4 is used on a plant comprising ALLELE2 or plant parts thereof, preferably for increasing yield.

In one embodiment at least one active ingredient selected from group I-5 is used on a plant comprising ALLELE2 or plant parts thereof, preferably for increasing yield.

In one embodiment at least one active ingredient selected from group I-6 is used on a plant comprising ALLELE2 or plant parts thereof, preferably for increasing yield.

In one embodiment at least one active ingredient selected from group F-1 is used on a plant comprising ALLELE2 or plant parts thereof, preferably for increasing yield.

In one embodiment at least one active ingredient selected from group F-2 is used on a plant comprising ALLELE2 or plant parts thereof, preferably for increasing yield.

In one embodiment at least one active ingredient selected from group F-3 is used on a plant comprising ALLELE2 or plant parts thereof, preferably for increasing yield.

In one embodiment at least one active ingredient selected from group F-4 is used on a plant comprising ALLELE2 or plant parts thereof, preferably for increasing yield.

In one embodiment at least one active ingredient selected from group F-5 is used on a plant comprising ALLELE2 or plant parts thereof, preferably for increasing yield.

In one embodiment at least one active ingredient selected from group F-6 is used on a plant comprising ALLELE2 or plant parts thereof, preferably for increasing yield.

In one embodiment at least one active ingredient selected from group SG-1 is used on a plant comprising ALLELE2 or plant parts thereof, preferably for increasing yield.

In one embodiment at least one active ingredient selected from group SG-2 is used on a plant comprising ALLELE2 or plant parts thereof, preferably for increasing yield.

In one embodiment at least one active ingredient selected from group SG-3 is used on a plant comprising ALLELE2 or plant parts thereof, preferably for increasing yield.

In one embodiment at least one active ingredient selected from group SG-4 is used on a plant comprising ALLELE2 or plant parts thereof, preferably for increasing yield.

In one embodiment at least one active ingredient selected from group SG-5 is used on a plant comprising ALLELE2 or plant parts thereof, preferably for increasing yield.

In one embodiment at least one active ingredient selected from group SG-6 is used on a plant comprising ALLELE2 or plant parts thereof, preferably for increasing yield.

In one embodiment at least one active ingredient selected from group SG-7 is used on a plant comprising ALLELE2 or plant parts thereof, preferably for increasing yield.

In one embodiment at least one active ingredient selected from group SG-8 is used on a plant comprising ALLELE2 or plant parts thereof, preferably for increasing yield.

In one embodiment at least one active ingredient selected from group H-1 is used on a plant comprising ALLELE3 or plant parts thereof, preferably for increasing yield.

In one embodiment at least one active ingredient selected from group H-2 is used on a plant comprising ALLELE3 or plant parts thereof, preferably for increasing yield.

In one embodiment at least one active ingredient selected from group H-3 is used on a plant comprising ALLELE3 or plant parts thereof, preferably for increasing yield.

In one embodiment at least one active ingredient selected from group H-4 is used on a plant comprising ALLELE3 or plant parts thereof, preferably for increasing yield.

In one embodiment at least one active ingredient selected from group H-5 is used on a plant comprising ALLELE3 or plant parts thereof, preferably for increasing yield.

In one embodiment at least one active ingredient selected from group P-1 is used on a plant comprising ALLELE3 or plant parts thereof, preferably for increasing yield.

In one embodiment at least one active ingredient selected from group SAF-1 is used on a plant comprising ALLELE3 or plant parts thereof, preferably for increasing yield.

In one embodiment at least one active ingredient selected from group SAF-2 is used on a plant comprising ALLELE3 or plant parts thereof, preferably for increasing yield.

In one embodiment at least one active ingredient selected from group SAF-3 is used on a plant comprising ALLELE3 or plant parts thereof, preferably for increasing yield.

In one embodiment at least one active ingredient selected from group SAF-4 is used on a plant comprising ALLELE3 or plant parts thereof, preferably for increasing yield.

In one embodiment at least one active ingredient selected from group B-1 is used on a plant comprising ALLELE3 or plant parts thereof, preferably for increasing yield.

In one embodiment at least one active ingredient selected from group B-2 is used on a plant comprising ALLELE3 or plant parts thereof, preferably for increasing yield.

In one embodiment at least one active ingredient selected from group B-3 is used on a plant comprising ALLELE3 or plant parts thereof, preferably for increasing yield.

In one embodiment at least one active ingredient selected from group B-4 is used on a plant comprising ALLELE3 or plant parts thereof, preferably for increasing yield.

In one embodiment at least one active ingredient selected from group B-5 is used on a plant comprising ALLELE3 or plant parts thereof, preferably for increasing yield.

In one embodiment at least one active ingredient selected from group I-1 is used on a plant comprising ALLELE3 or plant parts thereof, preferably for increasing yield.

In one embodiment at least one active ingredient selected from group I-2 is used on a plant comprising ALLELE3 or plant parts thereof, preferably for increasing yield.

In one embodiment at least one active ingredient selected from group I-3 is used on a plant comprising ALLELE3 or plant parts thereof, preferably for increasing yield.

In one embodiment at least one active ingredient selected from group I-4 is used on a plant comprising ALLELE3 or plant parts thereof, preferably for increasing yield.

In one embodiment at least one active ingredient selected from group I-5 is used on a plant comprising ALLELE3 or plant parts thereof, preferably for increasing yield.

In one embodiment at least one active ingredient selected from group I-6 is used on a plant comprising ALLELE3 or plant parts thereof, preferably for increasing yield.

In one embodiment at least one active ingredient selected from group F-1 is used on a plant comprising ALLELE3 or plant parts thereof, preferably for increasing yield.

In one embodiment at least one active ingredient selected from group F-2 is used on a plant comprising ALLELE3 or plant parts thereof, preferably for increasing yield.

In one embodiment at least one active ingredient selected from group F-3 is used on a plant comprising ALLELE3 or plant parts thereof, preferably for increasing yield.

In one embodiment at least one active ingredient selected from group F-4 is used on a plant comprising ALLELE3 or plant parts thereof, preferably for increasing yield.

In one embodiment at least one active ingredient selected from group F-5 is used on a plant comprising ALLELE3 or plant parts thereof, preferably for increasing yield.

In one embodiment at least one active ingredient selected from group F-6 is used on a plant comprising ALLELE3 or plant parts thereof, preferably for increasing yield.

In one embodiment at least one active ingredient selected from group SG-1 is used on a plant comprising ALLELE3 or plant parts thereof, preferably for increasing yield.

In one embodiment at least one active ingredient selected from group SG-2 is used on a plant comprising ALLELE3 or plant parts thereof, preferably for increasing yield.

In one embodiment at least one active ingredient selected from group SG-3 is used on a plant comprising ALLELE3 or plant parts thereof, preferably for increasing yield.

In one embodiment at least one active ingredient selected from group SG-4 is used on a plant comprising ALLELE3 or plant parts thereof, preferably for increasing yield.

In one embodiment at least one active ingredient selected from group SG-5 is used on a plant comprising ALLELE3 or plant parts thereof, preferably for increasing yield.

In one embodiment at least one active ingredient selected from group SG-6 is used on a plant comprising ALLELE3 or plant parts thereof, preferably for increasing yield.

In one embodiment at least one active ingredient selected from group SG-7 is used on a plant comprising ALLELE3 or plant parts thereof, preferably for increasing yield.

In one embodiment at least one active ingredient selected from group SG-8 is used on a plant comprising ALLELE3 or plant parts thereof, preferably for increasing yield.

In one embodiment at least one active ingredient selected from group H-1 is used on a plant comprising ALLELE4 or plant parts thereof, preferably for increasing yield.

In one embodiment at least one active ingredient selected from group H-2 is used on a plant comprising ALLELE4 or plant parts thereof, preferably for increasing yield.

In one embodiment at least one active ingredient selected from group H-3 is used on a plant comprising ALLELE4 or plant parts thereof, preferably for increasing yield.

In one embodiment at least one active ingredient selected from group H-4 is used on a plant comprising ALLELE4 or plant parts thereof, preferably for increasing yield.

In one embodiment at least one active ingredient selected from group H-5 is used on a plant comprising ALLELE4 or plant parts thereof, preferably for increasing yield.

In one embodiment at least one active ingredient selected from group P-1 is used on a plant comprising ALLELE4 or plant parts thereof, preferably for increasing yield.

In one embodiment at least one active ingredient selected from group SAF-1 is used on a plant comprising ALLELE4 or plant parts thereof, preferably for increasing yield.

In one embodiment at least one active ingredient selected from group SAF-2 is used on a plant comprising ALLELE4 or plant parts thereof, preferably for increasing yield.

In one embodiment at least one active ingredient selected from group SAF-3 is used on a plant comprising ALLELE4 or plant parts thereof, preferably for increasing yield.

In one embodiment at least one active ingredient selected from group SAF-4 is used on a plant comprising ALLELE4 or plant parts thereof, preferably for increasing yield.

In one embodiment at least one active ingredient selected from group B-1 is used on a plant comprising ALLELE4 or plant parts thereof, preferably for increasing yield.

In one embodiment at least one active ingredient selected from group B-2 is used on a plant comprising ALLELE4 or plant parts thereof, preferably for increasing yield.

In one embodiment at least one active ingredient selected from group B-3 is used on a plant comprising ALLELE4 or plant parts thereof, preferably for increasing yield.

In one embodiment at least one active ingredient selected from group B-4 is used on a plant comprising ALLELE4 or plant parts thereof, preferably for increasing yield.

In one embodiment at least one active ingredient selected from group B-5 is used on a plant comprising ALLELE4 or plant parts thereof, preferably for increasing yield.

In one embodiment at least one active ingredient selected from group I-1 is used on a plant comprising ALLELE4 or plant parts thereof, preferably for increasing yield.

In one embodiment at least one active ingredient selected from group I-2 is used on a plant comprising ALLELE4 or plant parts thereof, preferably for increasing yield.

In one embodiment at least one active ingredient selected from group I-3 is used on a plant comprising ALLELE4 or plant parts thereof, preferably for increasing yield.

In one embodiment at least one active ingredient selected from group I-4 is used on a plant comprising ALLELE4 or plant parts thereof, preferably for increasing yield.

In one embodiment at least one active ingredient selected from group I-5 is used on a plant comprising ALLELE4 or plant parts thereof, preferably for increasing yield.

In one embodiment at least one active ingredient selected from group I-6 is used on a plant comprising ALLELE4 or plant parts thereof, preferably for increasing yield.

In one embodiment at least one active ingredient selected from group F-1 is used on a plant comprising ALLELE4 or plant parts thereof, preferably for increasing yield.

In one embodiment at least one active ingredient selected from group F-2 is used on a plant comprising ALLELE4 or plant parts thereof, preferably for increasing yield.

In one embodiment at least one active ingredient selected from group F-3 is used on a plant comprising ALLELE4 or plant parts thereof, preferably for increasing yield.

In one embodiment at least one active ingredient selected from group F-4 is used on a plant comprising ALLELE4 or plant parts thereof, preferably for increasing yield.

In one embodiment at least one active ingredient selected from group F-5 is used on a plant comprising ALLELE4 or plant parts thereof, preferably for increasing yield.

In one embodiment at least one active ingredient selected from group F-6 is used on a plant comprising ALLELE4 or plant parts thereof, preferably for increasing yield.

In one embodiment at least one active ingredient selected from group SG-1 is used on a plant comprising ALLELE4 or plant parts thereof, preferably for increasing yield.

In one embodiment at least one active ingredient selected from group SG-2 is used on a plant comprising ALLELE4 or plant parts thereof, preferably for increasing yield.

In one embodiment at least one active ingredient selected from group SG-3 is used on a plant comprising ALLELE4 or plant parts thereof, preferably for increasing yield.

In one embodiment at least one active ingredient selected from group SG-4 is used on a plant comprising ALLELE4 or plant parts thereof, preferably for increasing yield.

In one embodiment at least one active ingredient selected from group SG-5 is used on a plant comprising ALLELE4 or plant parts thereof, preferably for increasing yield.

In one embodiment at least one active ingredient selected from group SG-6 is used on a plant comprising ALLELE4 or plant parts thereof, preferably for increasing yield.

In one embodiment at least one active ingredient selected from group SG-7 is used on a plant comprising ALLELE4 or plant parts thereof, preferably for increasing yield.

In one embodiment at least one active ingredient selected from group SG-8 is used on a plant comprising ALLELE4 or plant parts thereof, preferably for increasing yield.

In one embodiment at least one active ingredient selected from group H-1 is used on a plant comprising ALLELE5 or plant parts thereof, preferably for increasing yield.

In one embodiment at least one active ingredient selected from group H-2 is used on a plant comprising ALLELE5 or plant parts thereof, preferably for increasing yield.

In one embodiment at least one active ingredient selected from group H-3 is used on a plant comprising ALLELE5 or plant parts thereof, preferably for increasing yield.

In one embodiment at least one active ingredient selected from group H-4 is used on a plant comprising ALLELE5 or plant parts thereof, preferably for increasing yield.

In one embodiment at least one active ingredient selected from group H-5 is used on a plant comprising ALLELE5 or plant parts thereof, preferably for increasing yield.

In one embodiment at least one active ingredient selected from group P-1 is used on a plant comprising ALLELE5 or plant parts thereof, preferably for increasing yield.

In one embodiment at least one active ingredient selected from group SAF-1 is used on a plant comprising ALLELE5 or plant parts thereof, preferably for increasing yield.

In one embodiment at least one active ingredient selected from group SAF-2 is used on a plant comprising ALLELE5 or plant parts thereof, preferably for increasing yield.

In one embodiment at least one active ingredient selected from group SAF-3 is used on a plant comprising ALLELE5 or plant parts thereof, preferably for increasing yield.

In one embodiment at least one active ingredient selected from group SAF-4 is used on a plant comprising ALLELE5 or plant parts thereof, preferably for increasing yield.

In one embodiment at least one active ingredient selected from group B-1 is used on a plant comprising ALLELE5 or plant parts thereof, preferably for increasing yield.

In one embodiment at least one active ingredient selected from group B-2 is used on a plant comprising ALLELE5 or plant parts thereof, preferably for increasing yield.

In one embodiment at least one active ingredient selected from group B-3 is used on a plant comprising ALLELE5 or plant parts thereof, preferably for increasing yield.

In one embodiment at least one active ingredient selected from group B-4 is used on a plant comprising ALLELE5 or plant parts thereof, preferably for increasing yield.

In one embodiment at least one active ingredient selected from group B-5 is used on a plant comprising ALLELE5 or plant parts thereof, preferably for increasing yield.

In one embodiment at least one active ingredient selected from group I-1 is used on a plant comprising ALLELE5 or plant parts thereof, preferably for increasing yield.

In one embodiment at least one active ingredient selected from group I-2 is used on a plant comprising ALLELE5 or plant parts thereof, preferably for increasing yield.

In one embodiment at least one active ingredient selected from group I-3 is used on a plant comprising ALLELE5 or plant parts thereof, preferably for increasing yield.

In one embodiment at least one active ingredient selected from group I-4 is used on a plant comprising ALLELE5 or plant parts thereof, preferably for increasing yield.

In one embodiment at least one active ingredient selected from group I-5 is used on a plant comprising ALLELE5 or plant parts thereof, preferably for increasing yield.

In one embodiment at least one active ingredient selected from group I-6 is used on a plant comprising ALLELE5 or plant parts thereof, preferably for increasing yield.

In one embodiment at least one active ingredient selected from group F-1 is used on a plant comprising ALLELE5 or plant parts thereof, preferably for increasing yield.

In one embodiment at least one active ingredient selected from group F-2 is used on a plant comprising ALLELE5 or plant parts thereof, preferably for increasing yield.

In one embodiment at least one active ingredient selected from group F-3 is used on a plant comprising ALLELE5 or plant parts thereof, preferably for increasing yield.

In one embodiment at least one active ingredient selected from group F-4 is used on a plant comprising ALLELE5 or plant parts thereof, preferably for increasing yield.

In one embodiment at least one active ingredient selected from group F-5 is used on a plant comprising ALLELE5 or plant parts thereof, preferably for increasing yield.

In one embodiment at least one active ingredient selected from group F-6 is used on a plant comprising ALLELE5 or plant parts thereof, preferably for increasing yield.

In one embodiment at least one active ingredient selected from group SG-1 is used on a plant comprising ALLELE5 or plant parts thereof, preferably for increasing yield.

In one embodiment at least one active ingredient selected from group SG-2 is used on a plant comprising ALLELE5 or plant parts thereof, preferably for increasing yield.

In one embodiment at least one active ingredient selected from group SG-3 is used on a plant comprising ALLELE5 or plant parts thereof, preferably for increasing yield.

In one embodiment at least one active ingredient selected from group SG-4 is used on a plant comprising ALLELE5 or plant parts thereof, preferably for increasing yield.

In one embodiment at least one active ingredient selected from group SG-5 is used on a plant comprising ALLELE5 or plant parts thereof, preferably for increasing yield.

In one embodiment at least one active ingredient selected from group SG-6 is used on a plant comprising ALLELE5 or plant parts thereof, preferably for increasing yield.

In one embodiment at least one active ingredient selected from group SG-7 is used on a plant comprising ALLELE5 or plant parts thereof, preferably for increasing yield.

In one embodiment at least one active ingredient selected from group SG-8 is used on a plant comprising ALLELE5 or plant parts thereof, preferably for increasing yield.

### Uses

The treatment of the plants and plant parts with active ingredients or mixtures thereof is carried out directly or by acting on the environment, habitat or storage space using customary treatment methods, for example by dipping, spraying, atomizing, misting, evaporating, dusting, fogging, scattering, foaming, painting on, spreading, injecting, drenching, trickle irrigation and, in the case of propagation material, in particular in the case of seed, furthermore by the dry seed treatment method, the wet seed treatment method, the slurry treatment method, by encrusting, by coating with one or more coats and the like. It is furthermore possible to apply the active ingredients or mixtures thereof by the ultra-low volume method or to inject the active ingredient preparation or the active ingredient itself into the soil.

A preferred direct treatment of the plants is the leaf application treatment, i.e. active ingredients or mixtures thereof or compositions comprising active ingredients or mixtures thereof are applied to the foliage, it being possible for the treatment frequency and the application rate to be matched to achieve the yield increase in question.

In the case of systemically active compounds, active ingredients or mixtures thereof or compositions comprising active ingredients or mixtures thereof reach the plants via the root system. In this case, the treatment of the plants is effected by allowing active ingredients or mixtures thereof or compositions comprising active ingredients or mixtures thereof to act on the environment of the plant. This can be done for example by drenching, incorporating in the soil or into the nutrient solution, i.e. the location of the plant (for example the soil or hydroponic systems) is impregnated with a liquid form of active ingredients or mixtures thereof or compositions comprising active ingredients or mixtures thereof, or by soil application, i.e. the active ingredients or mixtures thereof or compositions comprising active ingredients or mixtures thereof are incorporated into the location of the plants in solid form (for example in the form of granules).

More particularly, the inventive use exhibits the advantages described on *Brassicaceae* plants, plant parts thereof, plant propagation material or the soil in which *Brassicaceae* plants are grown or intended to be grown in spray application, in seed treatment, in drip and drench applications, in-furrow application, chemigation, i.e. by addition of active ingredients or mixtures thereof to the irrigation water, and in hydroponic/mineral systems.

The combined use of active ingredients or mixtures thereof or compositions comprising active ingredients or mixtures thereof, with genetically modified cultivars, especially of transgenic oilseed rape cultivars, is additionally likewise possible.

### Application Rates

When using the active ingredients or mixtures thereof, the application rates can be varied within a relatively wide range, depending on the kind of application. The application rate of the inventive active ingredients is generally known to the skilled person. Generally, the following ranges can be applied
- in the case of treatment of plant parts, for example leaves: from 0.1 to 10 000 g/ha, preferably from 10 to 1000 g/ha, more preferably from 50 to 500 g/ha (in the case of application by watering or dripping, it is even possible to reduce the application rate, especially when inert substrates such as rockwool or perlite are used);
- in the case of seed treatment: from 0.1 to 200 g per 100 kg of seed, preferably from 1 to 150 g per 100 kg of seed, more preferably from 2.5 to 25 g per 100 kg of seed, even more preferably from 2.5 to 12.5 g per 100 kg of seed;
- in the case of soil treatment: from 0.1 to 10 000 g/ha, preferably from 1 to 5000 g/ha.

These application rates are merely by way of example and are not limiting for the purposes of the invention.

### Formulations

The present invention further relates to a composition for increasing yield, comprising at least one active ingredient or mixtures thereof. These are preferably fungicidal, bactericidal, herbicidal, insecticidal, acaricidal, or nematicidal compositions which comprise agriculturally suitable auxiliaries, solvents, carriers, surfactants or extenders.

According to the invention, a carrier is a natural or synthetic, organic or inorganic substance with which the active ingredients are mixed or combined for better applicability, in particular for application to plants or plant parts or seed. The carrier, which may be solid or liquid, is generally inert and should be suitable for use in agriculture.

Useful solid carriers include: for example ammonium salts and natural rock flours, such as kaolins, clays, talc, chalk, quartz, attapulgite, montmorillonite or diatomaceous earth, and synthetic rock flours, such as finely divided silica, alumina and silicates; useful solid carriers for granules include: for example, crushed and fractionated natural rocks such as calcite, marble, pumice, sepiolite and dolomite, and also synthetic granules of inorganic and organic flours, and granules of organic material such as paper, sawdust, coconut shells, maize cobs and tobacco stalks; useful emulsifiers and/or foam-formers include: for example nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP POE esters, alkylaryl and/or POP POE ethers, fat and/or POP POE adducts, POE- and/or POP-polyol derivatives, POE- and/or POP-sorbitan or -sugar adducts, alkyl or aryl sulphates, alkyl- or arylsulphonates and alkyl or aryl phosphates or the corresponding PO-ether adducts. Additionally suitable are oligo- or polymers, for example those derived from vinylic monomers, from acrylic acid, from EO and/or PO alone or in combination with, for example, (poly)alcohols or (poly)amines. It is also possible to use lignin and its sulphonic acid derivatives, unmodified and modified celluloses, aromatic and/or aliphatic sulphonic acids and also their adducts with formaldehyde.

The active ingredients or mixtures thereof can be converted to the customary formulations, such as solutions, emulsions, wettable powders, water- and oil-based suspensions, powders, dusts, pastes, soluble powders, soluble granules, granules for broadcasting, suspoemulsion concentrates, natural products impregnated with active ingredient, synthetic substances impregnated with active ingredients or mixtures thereof, fertilizers and also microencapsulations in polymeric substances.

The active ingredients or mixtures thereof can be applied as such, in the form of their formulations or the use forms prepared therefrom, such as ready-to-use solutions, emulsions, water- or oil-based suspensions, powders, wettable powders, pastes, soluble powders, dusts, soluble granules, granules for broadcasting, suspoemulsion concentrates, natural products impregnated with active ingredient, synthetic substances impregnated with active ingredient, fertilizers and also microencapsulations in polymeric substances. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

The formulations mentioned can be prepared in a manner known per se, for example by mixing the active ingredients or mixtures thereof with at least one customary extender, solvent or diluent, emulsifier, dispersant and/or binder or fixing agent, wetting agent, a water repellent, if appropriate siccatives and UV stabilizers and if appropriate dyes and pigments, antifoams, preservatives, secondary thickeners, stickers, gibberellins and also other processing auxiliaries.

The present invention includes not only formulations which are already ready for use and can be deployed with a suitable apparatus to the plant or the seed, but also commercial concentrates which have to be diluted with water prior to use. active ingredients or mixtures thereof may be present as such or in their (commercial) formulations and in the use forms prepared from these formulations as a mixture with other (known) active ingredients, such as insecticides, attractants, sterilants, bactericides, acaricides, nematicides, fungicides, growth regulators, herbicides, fertilizers, safeners and/or semiochemicals.

The auxiliaries used may be those substances which are suitable for imparting particular properties to the composition itself or and/or to preparations derived therefrom (for example spray liquors, seed dressings), such as certain technical properties and/or also particular biological properties. Typical auxiliaries include: extenders, solvents and carriers.

Suitable extenders are, for example, water, polar and nonpolar organic chemical liquids, for example from the classes of the aromatic and nonaromatic hydrocarbons (such as paraffins, alkylbenzenes, alkylnaphthalenes, chlorobenzenes), the alcohols and polyols (which may optionally also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

Liquefied gaseous extenders or carriers are understood to mean liquids which are gaseous at standard temperature and under standard pressure, for example aerosol propellants such as halohydrocarbons, or else butane, propane, nitrogen and carbon dioxide.

In the formulations it is possible to use tackifiers such as carboxymethylcellulose, natural and synthetic polymers in the form of powders, granules or latices, such as gum arabic, polyvinyl alcohol and polyvinyl acetate, or else natural phospholipids such as cephalins and lecithins and synthetic phospholipids. Further additives may be mineral and vegetable oils.

If the extender used is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example petroleum fractions, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, or else water.

Compositions comprising active ingredients or mixtures thereof may additionally comprise further components, for example surfactants. Suitable surfactants are emulsifiers and/or foam formers, dispersants or wetting agents having ionic or nonionic properties, or mixtures of these surfactants. Examples thereof are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. The presence of a surfactant is necessary if one of the active ingredients and/or one of the inert carriers is insoluble in water and when application is effected in water. The proportion of surfactants is between 5 and 40 per cent by weight of the inventive composition.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Further additives may be perfumes, mineral or vegetable, optionally modified oils, waxes and nutrients (including trace nutrients), such as salts of iron, manganese, boron, copper, cobalt, molybdenum and zinc.

Additional components may be stabilizers, such as cold stabilizers, preservatives, antioxidants, light stabilizers, or other agents which improve chemical and/or physical stability.

If appropriate, other additional components may also be present, for example protective colloids, binders, adhesives, thickeners, thixotropic substances, penetrants, stabilizers, sequestering agents, complex formers. In general, the active ingredients or mixtures thereof can be combined with any solid or liquid additive commonly used for formulation purposes.

The formulations contain generally between 0.05 and 99% by weight, 0.01 and 98% by weight, preferably between 0.1 and 95% by weight, more preferably between 0.5 and 90% of active ingredients or mixtures thereof, most preferably between 10 and 70 per cent by weight.

The formulations described above can be used for increasing yield, in which the compositions comprising compounds of the formula (I) are applied to the *Brassicaceae* and/or in their habitat.

### Plants

According to the invention all plants and plant parts can be treated. By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods. By plant parts is meant all above ground and below ground parts and organs of plants such as shoot, leaf, blossom and root, whereby for example leaves, needles, stems, branches, blossoms, fruiting bodies, fruits and seed as well as roots, corms and rhizomes are listed. Crops and vegetative and generative propagating material, for example cuttings, corms, rhizomes, runners, slips and seeds also belong to plant parts. Seeds are preferred plant parts.

In one embodiment crop plants belonging to the plant family Brassicaceae are Brassica plants.

In a preferred embodiment crop species, cultivars and varieties belonging to the plant genus Brassica are
- *Brassica carinata*: Abyssinian mustard or Abyssinian cabbage
- *Brassica elongate*: elongated mustard
- *Brassica fruticulosa*: Mediterranean cabbage
- *Brassica juncea*: Indian mustard, brown and leaf mustards, Sarepta mustard
- *Brassica napus* comprising winter rapeseed, spring rapeseed, rutabaga (*Brassica napus* subsp *rapifera* swede/Swedish turnip/swede turnip)
- *Brassica narinosa*: broadbeaked mustard
- *Brassica nigra*: black mustard
- *Brassica oleracea* comprising cultivars like kale, cabbage, broccoli, cauliflower, kai-lan, Brussels sprouts, kohlrabi
- *Brassica perviridis*: tender green, mustard spinach
- *Brassica rapa* (syn B. c*ampestris*) comprising Chinese cabbage, turnip, rapini, komatsuna
- *Brassica rupestris*: brown mustard
- *Brassica septiceps:* seventop turnip
- *Brassica tournefortii*: Asian mustard
- *Brassica alba* (syn *Sinapis alba*, white mustard)
- Canola varieties

To use the name canola, an oilseed plant must meet the following internationally regulated standard:
"Seeds of the genus Brassica (*Brassica napus*, *Brassica rapa* or *Brassica juncea*) from which the oil shall contain less than 2% erucic acid in its fatty acid profile and the solid component shall contain less than 30 micromoles of any one or any mixture of 3-butenyl glucosinolate, 4-pentenyl glucosinolate, 2-hydroxy-3 butenyl glucosinolate, and 2-hydroxy- 4-pentenyl glucosinolate per gram of air-dry, oil-free solid. "

Further preferred crop plants belonging to the plant family *Brassicaceae* are horseradish (Armoracia rusticana), radish (e.g. *Raphanus sativus* var. *oleiformis*, *Raphanus sativus* L. var. *sativus*.

More preferred Brassica plants, plant parts including seeds according to the present invention are oilseed rape plants, plant parts including seeds (*Brassica napus*), Canola plants, plant parts including seeds or *Brassica juncea* plants, plant parts including seeds; more preferred winter oilseed rape plants, plant parts including seeds (*Brassica napus*), spring oilseed rape plants, plant parts including seeds or Canola, plant parts including seeds.

In one aspect *Brassica napus* or *juncea* plants, plant parts or seeds are hybrid plants, plant parts including seeds. In another aspect *Brassica napus* or *juncea* hybrids are Ogura hybrids, Ms8/Rf3 hybrids (marketed under the tradename Invigor) or Ms11/Rf3 hybrids.

In another embodiment the *Brassica napus* or *juncea* plants, plant parts including seeds are tolerant to one or more of the herbicides selected from the group of glufosinate, glyphosate (tradename RoundupReady), imazamethabenz, imazamethabenz-methyl, imazamox, imazamox-ammonium, imazapic, imazapic-ammonium, imazapyr, imazapyr-isopropyl-ammonium, imazaquin, imazaquin-ammonium, imazethapyr, imazethapyr-ammonium, atrazine, simazine.

The term "growth stage" refers to the growth stages as defined by the BBCH Codes in "Growth stages of mono- and dicotyledonous plants", 2nd edition 2001, edited by Uwe Meier from the Federal Biological Research Centre for Agriculture and Forestry. The BBCH codes are a well-established system for a uniform coding of phonologically similar growth stages of all mono- and dicotyledonous plant species. The abbreviation BBCH derives from "Biologische Bundesanstalt, Bundessortenamt und Chemische Industrie".

Some of these BBCH growth stages and BBCH codes for oilseed rape plants are indicated in the following.
Growth stage 1: Leaf development 1
BBCH 10 - Cotyledons completely unfolded
BBCH 11 - First leaf unfolded
BBCH 12 - 2nd leaf unfolded
BBCH 13 - 3rd leaf unfolded
BBCH 14 - 18 Stages continuous till ... (4 - 8th leaf unfolded)
BBCH 19 - 9 or more leaves unfolded
Growth stage 2: Formation of side shoots
BBCH 20 - No side shoots
BBCH 21 - Beginning of side shoot development: first side shoot detectable
BBCH 22 - 2nd side shoots detectable
BBCH 23 - 3rd side shoots detectable
BBCH 24 - 4th side shoots detectable
BBCH 25 - 5th side shoots detectable
BBCH 26 - 28 - Stages continuous till ... (6 - 8 side shoots detectable)
BBCH 29 End of side shoot development: 9 or more side shoots detectable
Growth stage 3: Stem elongation2
BBCH 30 - Beginning of stem elongation: no internodes ("rosette")
BBCH 31 - 1 visibly extended internode
BBCH 32 - 2nd visibly extended internode
BBCH 33 - 3rd visibly extended internode
BBCH 39 - 9 or more visibly extended internodes
Growth stage 8: Ripening
BBCH 80 - Beginning of ripening: seed green, filling pod cavity
BBCH 81 - 10% of pods ripe, seeds dark and hard
BBCH 82 - 20% of pods ripe, seeds dark and hard
BBCH 83 - 30% of pods ripe, seeds dark and hard
BBCH 84 - 40% of pods ripe, seeds dark and hard
BBCH 85 - 50% of pods ripe, seeds dark and hard
BBCH 86 - 60% of pods ripe, seeds dark and hard
BBCH 87 - 70% of pods ripe, seeds dark and hard
BBCH 88 - 80% of pods ripe, seeds dark and hard
BBCH 89 - Fully ripe: nearly all pods ripe, seeds dark and hard
Growth stage 9: Senescence
BBCH 97- Plant dead and dry
BBCH 99 - Harvested product

Particular preference is given in accordance with the invention to treating plants of the plant cultivars which are each commercially available or in use. Plant cultivars are understood to mean plants which have new properties ("traits") and which have been obtained by conventional breeding, by mutagenesis or with the aid of recombinant DNA techniques. Crop plants may accordingly be plants which can be obtained by conventional breeding and optimization methods or by biotechnology and genetic engineering methods or combinations of these methods, including the transgenic plants and including the plant varieties which can and cannot be protected by plant variety rights.

The method according to the invention can thus also be used for the treatment of genetically modified organisms (GMOs), for example plants or seeds. Genetically modified plants (or transgenic plants) are plants in which a heterologous gene has been integrated stably into the genome. The term "heterologous gene" means essentially a gene which is provided or assembled outside the plant and which, on introduction into the cell nucleus genome, imparts new or improved agronomic or other properties to the chloroplast genome or the mitochondrial genome of the transformed plant by virtue of it expressing a protein or polypeptide of interest or by virtue of another gene which is present in the plant, or other genes which are present in the plant, being downregulated or silenced (for example by means of antisense technology, co-suppression technology or RNAi technology [RNA interference]). A heterologous gene present in the genome is likewise referred to as a transgene. A transgene which is defined by its specific presence in the plant genome is referred to as a transformation or transgenic event.

Plants and plant cultivars which are preferably treated according to the invention include all plants which have genetic material which imparts particularly advantageous, useful traits to these plants (whether obtained by breeding and/or biotechnological means).

### Seed Treatment

The treatment of the seed of plants has been known for a long time and is the subject of constant improvements. Nevertheless, the treatment of seed gives rise to a series of problems which cannot always be solved in a satisfactory manner. For instance, it is desirable to develop methods for protecting the seed, the germinating plant and the resulting plants or plant parts, which dispense with, or at least significantly reduce, the additional deployment of crop protection products after planting or after emergence of the plants. It is additionally desirable to optimize the amount of active ingredients or mixtures thereof used in such a way as to provide the best possible protection for the seed and the germinating plant in order to achieve the yield increase, but without damaging the Brassicaceae plant itself by the active ingredient used.

The present invention therefore relates more particularly also to a method for treating seed to increase yield in Brassicaceae plants which grow from the seed or seedlings, by treating the Brassicaceae seed with active ingredients or mixtures thereof.

In another embodiment a method for treating seed to increase yield in Brassicaceae plants at BBCH stage 10 or later which grow from the seed or seedlings, by treating the Brassicaceae seed at BBCH stage 00 with active ingredients or mixtures thereof.

In another embodiment a method for treating seed to increase yield in Brassica napus plants at BBCH stage 10 or later which grow from the seed or seedlings, by treating the Brassica napus seed at BBCH stage 00 with active ingredients or mixtures thereof.

In another embodiment a method for treating seed to increase yield in canola plants at BBCH stage 10 or later which grow from the seed or seedlings, by treating the canola seed at BBCH stage 00 with active ingredients or mixtures thereof.

In another embodiment a method for treating seed to increase yield in canola hybrid plants at BBCH stage 10 or later which grow from the seed or seedlings, by treating the canola hybrid seed at BBCH stage 00 with active ingredients or mixtures thereof.

In another embodiment a method for treating seed to increase yield in herbicide tolerant canola hybrid plants at BBCH stage 10 or later which grow from the seed or seedlings, by treating the herbicide tolerant canola hybrid seed at BBCH stage 00 with active ingredients or mixtures thereof.

The invention likewise relates to the use of active ingredients or mixtures for treatment of seed to increase yield in the the plants or those plant parts which grow from the seeds.

It is likewise considered to be advantageous that active ingredients or mixtures thereof, can especially also be used in transgenic seed.

The active ingredients or mixtures thereof are applied to the seed alone or in a suitable formulation. Preferably, the seed is treated in a state in which it is stable enough to avoid damage during treatment. In general, the seed may be treated at any time between harvest and sowing. The seed typically used has been separated from the plant and freed from cobs, shells, stalks, coats, hairs or the fruit flesh. For example, it is possible to use seed which has been harvested, cleaned and dried to a moisture content of less than 15% by weight. Alternatively, it is also possible to use seed which, after drying, for example, has been treated with water and then dried again.

When treating the seed, it must generally be ensured that the amount of active ingredients or mixtures thereof applied to the seed and/or of further additives is selected such that the germination of the seed is not impaired, and that the resulting plant is not damaged. This should be noted in particular in the case of active ingredients which can have phytotoxic effects at particular application rates.

The active ingredients or mixtures thereof can be applied directly, i.e. without containing any further components and without having been diluted. In general, it is preferable to apply active ingredients or mixtures thereof, to the seed in the form of a suitable formulation. Suitable formulations and methods for seed treatment are known to those skilled in the art and are described, for example, in the following documents: US 4,272,417 A, US 4,245,432 A, US 4,808,430 A, US 5,876,739 A, US 2003/0176428 A1, WO 2002/080675 A1, WO 2002/028186 A2.

The active ingredients or mixtures thereof can be converted to the customary seed dressing formulations, such as solutions, emulsions, suspensions, powders, foams, slurries or other coating materials for seed, and also ULV formulations.

These formulations are produced in a known manner, by mixing the active ingredients or active ingredient combinations with customary additives, for example customary extenders and solvents or diluents, dyes, wetting agents, dispersants, emulsifiers, defoamers, preservatives, secondary thickeners, stickers, gibberellins and also water.

Useful dyes which may be present in the seed dressing formulations usable in accordance with the invention are all dyes customary for such purposes. It is possible to use both sparingly water-soluble pigments and water-soluble dyes. Examples include the dyes known under the Rhodamine B, C.I. Pigment Red 112 and C.I. Solvent Red 1 names.

The wetting agents which may be present in the seed dressing formulations usable in accordance with the invention include all substances which promote wetting and are customary for formulation of active agrochemical ingredients. Usable with preference are alkyl naphthalenesulphonates, such as diisopropyl or diisobutyl naphthalenesulphonate.

The dispersants and/or emulsifiers which may be present in the seed dressing formulations usable in accordance with the invention include all nonionic, anionic and cationic disperants which are customary for formulation of active agrochemical ingredients. Usable with preference are nonionic or anionic dispersants or mixtures of nonionic or anionic dispersants. Suitable nonionic dispersants include especially ethylene oxide-propylene oxide block polymers, alkylphenol polyglycol ethers and tristyrylphenol polyglycol ethers, and the phosphated or sulphated derivatives thereof. Suitable anionic dispersants are especially lignosulphonates, polyacrylic acid salts and arylsulphonate-formaldehyde condensates.

The defoamers which may be present in the seed dressing formulations usable in accordance with the invention include all foam-inhibiting substances customary for formulation of active agrochemical ingredients. Usable with preference are silicone defoamers and magnesium stearate.

The preservatives which may be present in the seed dressing formulations usable in accordance with the invention include all substances usable for such purposes in agrochemical formulations. Examples include dichlorophene and benzyl alcohol hemiformal.

Useful secondary thickeners which may be present in the seed dressing formulations usable in accordance with the invention include all substances usable for such purposes in agrochemical formulations. Preferred examples include cellulose derivatives, acrylic acid derivatives, xanthan, modified clays and finely divided silica.

Useful stickers which may be present in the seed dressing formulations usable in accordance with the invention are all customary binders usable in seed dressing compositions. Preferred examples include polyvinylpyrrolidone, polyvinyl acetate, polyvinyl alcohol and tylose.

The gibberellins which may be present in the seed dressing formulations usable in accordance with the invention are preferably gibberellins A1, A3 (= gibberellic acid), A4 and A7, particular preference being given to using gibberellic acid. The gibberellins are known (cf. R. Wegler "Chemie der Pflanzenschutz- und Schädlingsbekämpfungsmittel" [Chemistry of Crop Protection and Pest Control Compositions], vol. 2, Springer Verlag, 1970, p. 401-412).

The seed dressing formulations usable in accordance with the invention can be used to treat either directly or after preceding dilution with water. The seed dressing preparations usable in accordance with the invention or the dilute preparations thereof can also be used to dress seed of transgenic plants. In this case, it is also possible for additional synergistic effects to occur in interaction with substances formed by expression.

For treatment of seed with the seed dressing formulations usable in accordance with the invention, or the preparations prepared therefrom by adding water, all mixing units usable customarily for the seed dressing are useful. Specifically, the seed dressing procedure is to introduce the seed into a mixer, to add the particular desired amount of seed dressing formulations, either as such or after preceding dilution with water, and to mix until the formulation is distributed homogeneously on the seed. This may be followed by a drying operation.

The application rate of seed dressing formulations usable in accordance with the invention may vary within a relatively wide range. It is guided by the particular content of the active ingredients in the formulations and by the seed. The application rates of seed treatment compositions comprising active ingredients or mixtures thereof are: from 0.1 to 200 g per 100 kg of seed, preferably from 1 to 150 g per 100 kg of seed, more preferably from 2.5 to 25 g per 100 kg of seed, even more preferably from 2.5 to 12.5 g per 100 kg of seed;

The active ingredients or mixtures thereof, may be present in their commercially available formulations and in the use forms, prepared from these formulations, as a mixture with other active ingredients, such as insecticides, attractants, sterilants, bactericides, acaricides, nematicides, fungicides, growth regulators, herbicides, safeners, fertilizers or semiochemicals.

In another embodiment the active ingredients may be present in their commercially available formulations and in the use forms, prepared from these formulations, as a mixture with one or more active ingredients.

The example which follows serves to illustrate the invention, but without restricting it.

**LIST OF ABBREVIATIONS**

| | | |
|---|---|---|
| 2mepsps | Glyphosate oxidase from Ochrobactrum anthropi strain LBAA | decreases binding affinity for glyphosate, thereby increasing tolerance to glyphosate herbicide |
| accdPc | 1-amino-cyclopropane-1-carboxylic acid deaminase enzyme from *Pseudomonas chloroaphis* | metabolizes the precursor of the fruit ripening hormone ethylene, resulting in delayed fruit ripening |
| antisense Pg | no functional polygalacturonase enzyme is produced (transcription of the endogenous enzyme is suppressed by a gene silencing mechanism) | inhibits the production of polygalacturonase enzyme responsible for the breakdown of pectin molecules in the cell wall, and thus causes delayed softening of the fruit |
| athb17 | a protein from *Arabidopsis thaliana* of the class II family of the homeodomain-leucine zipper (HD-Zip) transcription factors. | Modulates plant growth and development and regulates gene expression |
| avhppd-03 | p-hydroxyphenylpyruvate dioxygenase from *Avena sativa* | Tolerance to Mesotrione herbicide |
| bar | phosphinothricin N-acetyltransferase (PAT) enzyme from *Streptomyces hygroscopicus* | eliminates herbicidal activity of glufosinate (phosphinothricin) herbicides by acetylation |
| bamase | barnase ribonuclease enzyme from *Bacillus amyloliquefaciens* | causes male sterility by interfering with RNA production in the tapetum cells of the anther |
| barstar | barnase ribonuclease inhibitor from *Bacillus amyloliquefaciens* | restores fertility by repressing the inhibitory effect of bamase on tapetum cells of the anther |
| bbx32 | protein from *Arabidopsis thaliana* that interacts with one or more endogenous transcription factors to regulate the plant's day/night physiological processes | to modulate plant's diurnal biology and to enhance growth and reproductive development |
| bxn | nitrilase enzyme from *Klebsiella pneumoniae subsp. Ozaenae* | eliminates herbicidal activity of oxynil herbicides (eg. bromoxynil) |
| ccomt (inverted repeat) | dsRNA that suppresses endogenous S-adenosyl-L-methionine: trans-caffeoyl CoA 3-O-methyltransferase (CCOMT gene) RNA transcript levels via the RNA interference (RNAi) pathway | reduces content of guaiacyl (G) lignin |
| CordapA | dihydrodipicolinate synthase enzyme from Corynebacterium glutamicum | increases the production of amino acid lysine |
| cp4 epsps | herbicide tolerant form of 5-enolpyruvulshikimate-3-phosphate synthase (EPSPS) enzyme from | decreases binding affinity for glyphosate, thereby conferring increased tolerance to glyphosate herbicide |
| | Agrobacterium tumefaciens strain CP4 | |
| cry1A.105 | Cry1A.105 protein which comprises the Cry1Ab, Cry1F and Cry1Ac proteins from Bacillus thuringiensis subsp. Kumamotoensis | confers resistance to lepidopteran insects by selectively damaging their midgut lining |
| cry1Ab | Cry1Ab delta-endotoxin from *Bacillus thuringiensis subsp. Kurstaki* | confers resistance to lepidopteran insects by selectively damaging their midgut lining |
| cry1Ac | Cry1Ac delta-endotoxin from *Bacillus thuringiensis subsp. Kurstaki strain HD73* | confers resistance to lepidopteran insects by selectively damaging their midgut lining |
| CrylF | CrylF delta-endotoxin from *Bacillus thuringiensis var*. *Aizawai* | confers resistance to lepidopteran insects by selectively damaging their midgut lining |
| crylFa2 | synthetic form of cry1F gene derived from *Bacillus thuringiensis var. Aizawai* | confers resistance to lepidopteran insects by selectively damaging their midgut lining |
| cry2Ab2 | Cry2Ab delta-endotoxin from *Bacillus thuringiensis subsp. Kumamotoensis* | confers resistance to lepidopteran insects by selectively damaging their midgut lining |
| cry2Ae | Cry2Ae delta-endotoxin from *Bacillus thuringiensis subsp Dakota* | confers resistance to lepidopteran insects by selectively damaging their midgut lining |
| cry34Ab1 | Cry34Ab1 delta-endotoxin from *Bacillus thuringiensis strain PS149B1* | confers resistance to coleopteran insects particularly com rootworm by selectively damaging their midgut lining |
| cry35Ab1 | Cry35Ab1 delta-endotoxin from *Bacillus thuringiensis strain PS149B1* | confers resistance to coleopteran insects particularly com rootworm by selectively damaging their midgut lining |
| cry3A | cry3A delta endotoxin from *Bacillus thuringiensis subsp. Tenebrionis* | confers resistance to coleopteran insects by selectively damaging their midgut lining |
| cry3Bb1 | Cry3Bbl delta endotoxin from *Bacillus thuringiensis subsp. Kumamotoensis* | confers resistance to coleopteran insects particularly corn rootworm by selectively damaging their midgut lining |
| cspB | cold shock protein B from *Bacillus subtilis* | maintains normal cellular functions under water stress conditions by preserving RNA stability and translation |
| Dgat2A | diacylglycerol acyltransferase type 2A from *Mortierella ramanniana* | modifies oil profile |
| dmo | dicamba mono-oxygenase enzyme from *Stenotrophomonas maltophilia strain DI-6* | confers tolerance to the herbicide dicamba (2-methoxy-3,6-dichlorobenzoic acid) by using dicamba as substrate in an enzymatic reaction |
| dvsnf7 (inverted | double-stranded RNA transcript containing a 240 bp fragment of the Western Com | RNAi interference resulting to down-regulation of the function of the targeted Snf7 gene leading |
| repeat) | Rootworm Snf7 gene | to Western Com Rootworm mortality. |
| epsps grg23 ace 5 | modified 5-enolpyruvylshikimate-3-phosphate synthase (EPSPS) protein or EPSPS ACE5 protein from *Arthrobacter globiformis* | confers tolerance to glyphosate herbicides |
| fad2-1A (sense antisense) | no functional enzyme is produced (production of delta-12 desaturase enzyme is suppressed by RNA interference) | reduces desaturation of 18:1 oleic acid to 18:2 linoleic acid; increases the levels of monounsaturated oleic acid and decreases the levels of saturated linoleic acid in the seed |
| fatb1-A (sense antisense) | no functional enzyme is produced (production of FATB enzymes or acyl-acyl carrier protein thioesterases is suppressed by RNA interference) | decreases the transport of saturated fatty acids out of the plastid, thereby increasing their availability to desaturation to 18:1 oleic acid; reduces the levels of saturated fatty acids and increases the levels of 18:1 oleic acid |
| goxv247 | glyphosate oxidase from *Ochrobactrum anthropi strain LBAA* | confers tolerance to glyphosate herbicides by degrading glyphosate into aminomethylphosphonic acid (AMPA) and glyoxylate |
| hppdPF W336 | modified p-hydroxyphenylpyruvate dioxygenase (hppd) enzyme from *Pseudomonas fluorescens* strain A32 | confers tolerance to HPPD-inhibiting herbicides (such as isoxaflutole) by reducing the specificity for the herbicide's bioactive constituent |
| Nc.Fad3 | delta 15 desaturase protein from *Neurospora crassa* | desaturates certain endogenous fatty acids resulting in the production of stearidonic acid (SDA), an omega-3 fatty acid |
| nptII | neomycin phosphotransferase II enzyme | allows transformed plants to metabolize neomycin and kanamycin antibiotics during selection |
| pat | phosphinothricin N-acetyltransferase (PAT) enzyme - synthetic form of pat gene derived from *Streptomyces viridochromogenes strain Tu 494* | eliminates herbicidal activity of glufosinate (phosphinothricin) herbicides by acetylation |
| pinII | protease inhibitor protein from *Solanum tuberosum* | enhances defense against insect predators by reducing the digestibility and nutritional quality of the leaves |
| Pj.D6D | delta 6 desaturase protein from *Primula juliae* | desaturates certain endogenous fatty acids resulting in the production of stearidonic acid (SDA), an omega-3 fatty acid |
| PLRV orf1 | putative replicase domain of the potato leaf roll virus (PLRV) | confers resistance to potato leaf roll virus (PLRV) through gene silencing mechanism |
| PLRV orf2 | putative helicase domain of the potato leaf roll virus (PLRV) | confers resistance to potato leaf roll virus (PLRV) through gene silencing mechanism |
| PVY coat protein | coat protein of the potato virus Y (PVY | confers resistance to potato virus Y (PVY) through "pathogen-derived resistance" mechanism |
| te | 12:0 ACP thioesterase enzyme from *Umbellularia californica* (bay leaf) | increases the level of triacylglycerides containing esterified lauric acid (12:0) |
| uidA | beta-D-glucuronidase (GUS) enzyme from *Escherichia coli* | produces blue stain on treated transformed tissue, which allows visual selection |
| vip3A | VIP3A vegetative insecticidal protein from *Bacillus thuringiensis strain AB88* | confers resistance to feeding damage caused by lepidopteran insects by selectively damaging their midgut lining |

## Claims

1. Method for increasing yield in *Brassicaceae* comprising at least one event or allele according table A, wherein the *Brassicaceae* plants, plant parts thereof or the soil in which *Brassicaceae* plants are grown or intended to be grown are treated with the active ingredients or mixtures thereof.

2. Method according to Claims 1, wherein the active ingredients or mixtures thereof are applied as a foliar treatment to *Brassicaceae* plants, as a seed treatment to *Brassicaceae* seeds or as a soil application to the habitat where *Brassicaceae* plants are grown or will be intended to grow.

3. Method according to Claim 1 or 2 wherein the active ingredients or mixtures thereof are selected from the group H-1, H-2, H-3, H-4, H-5, P-1, SAF-1, SAF-2, SAF-3, SAF-4, B-1, B-2, B-3, B-4, B-5, I-1, I-2,1-3,1-4,1-5,1-6, F-1, F-2, F-3, F-4, F-5, F-6, SG-1, SG-2, SG-3, SG-4, SG-5, SG-6, SG-7, SG-8.

4. Method according to any of Claims 1 to 3, wherein the events or alleles are selected from the group consisting of 23-18-17, MON88302, RT200, RT73, MS8, RF3, POSH122, MS11, Ogura, R113, R40, R2000.

5. Method according to any of Claims 1 to 4, wherein that the *Brassicaceae* are hybrid winter oilseed rape or hybrid spring oilseed rape.

6. Method according to any of Claims 1 to 5, wherein that the *Brassicaceae* are hybrid Canola.
